# EUROPEAN PATENT SPECIFICATION

(11) **EP 3 298 000 B1**
(45) Date of publication and mention of the grant of the patent: **24.04.2019**
(21) Application number: 16724389.8
(22) Date of filing: 19.05.2016
(51) Int. Cl.: C07D 401/12, C07D 409/12, C07D 409/14, C07D 333/12, C07D 333/16, C07D 333/20, C07D 333/22, C07D 333/24, A01N 43/56

(54) **FUNGICIDAL N-CYCLOALKYL-N-{[ORTHO-(1-SUBSTITUTED-CYCLOALKYL)HETEROARYL]METHYL}PYRAZOLE(THIO)CARBOXAMIDES**
FUNGIZIDE N-CYCLOALKYL-N-{[ORTHO-(1-SUBSTITUIERTES-CYCLOALKYL)HETEROARYL]METHYL}PYRAZOLE(THIO)CARBOXAMIDE
N-CYCLOALKYL-N-{[ORTHO-(CYCLOALKYL-1-SUBSTITUÉ)HÉTÉROARYL]MÉTHYL}PYRAZOLE(THIO)CARBOXAMIDES FONGICIDES

(30) Priority: 21.05.2015 EP 15290133
(43) Date of publication of application: 28.03.2018
(73) Proprietor: Bayer CropScience Aktiengesellschaft, 40789 Monheim am Rhein (DE)
(72) Inventor: CRISTAU, Pierre, 69009 Lyon (FR); DESBORDES, Philippe, 69006 Lyon (FR); GEIST, Julie, 69009 Lyon (FR); NICOLAS, Lionel, 69001 Lyon (FR); RINOLFI, Philippe, 69380 Châtillon d'Azergues (FR); SCHMIDT, Jan Peter, Folsom, CA 95630 (US); TSUCHIYA, Tomoki, 69001 Lyon (FR)
(74) Representative: Guitton, Carole
(86) International application number: PCT/EP2016/061202
(87) International publication number: WO 2016/184941

(56) References cited:
- WO-A1-2012/059497
- WO-A1-2015/082586
- WO-A2-2010/130767
- US-A1- 2004 209 921
- HENRI RUDLER ET AL: "Bis(trimethylsilyl)ketene acetals as C,O-dinucleophiles: one-pot formation of polycyclic [gamma]- and [delta]-lactones from pyridines and pyrazines", EUROPEAN JOURNAL OF ORGANIC CHEMISTRY, vol. 2005, no. 17, 2005, pages 3724-3744, XP055206759, ISSN: 1434-193X, DOI: 10.1002/ejoc.200500162
- ALEJANDRO RIVERA-HERNÁNDEZ ET AL: "Reactivity of pyridines bearing EWG with bis-(TMS)ketene acetals. Substituent-induced lactonization reaction", TETRAHEDRON, vol. 70, no. 10, 2014, pages 1861-1871, XP028614755, ISSN: 0040-4020, DOI: 10.1016/J.TET.2014.01.044
- DATABASE CAPLUS [Online] CHEMICAL ABSTRACTS SERVICE, COLUMBUS, OHIO, US; 1963, Carlo Giulio Casinovi et al: "Bitter substances contained in Ailanthus altissima", XP002758886, retrieved from STN Database accession no. 1964:461786

## Description

The present invention relates to fungicidal N-cycloalkyl-N-{[ortho-(1-substitutedcycloalkyl)heteroaryl]-methylene} carboxamide derivatives and their thiocarbonyl derivatives, their process of preparation and intermediate compounds for their preparation, their use as fungicides, particularly in the form of fungicidal compositions and methods for the control of phytopathogenic fungi of plants, using these compounds or their compositions.

In international patent application WO-2006/120224 certain N-cycloalkyl-N-(pyridylmethyl)carboxamides are generically embraced in a broad disclosure of numerous compounds of the following formula: 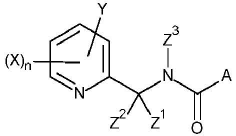 wherein A represents a carbo-linked, unsaturated, 5-membered heterocyclyl group, Z³ represents a substituted or non-substituted C₃-C₇-cycloalkyl group, Y represents a haloalkyl group and X can represent various substituents among which an unsubstituted C₃-C₇-cycloalkyl. However, there is no explicit disclosure or suggestion to select in this document of any such derivative wherein X can represent a substituted C₃-C₇-cycloalkyl.

In international patent application WO-2008/015189 certain N-cycloalkyl-N-(heteroarylmethyl) (thio)carboxamides are generically embraced in a broad disclosure of numerous compounds of the following formula: 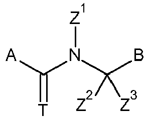 wherein A represents a carbo-linked, unsaturated, 5-membered heterocyclyl group, T can represent O or S, Z¹ represents a substituted or non-substituted C₃-C₇-cycloalkyl group, B represents a carbo-linked, unsaturated, 5-membered heterocyclyl group that can be substituted by up to four groups X, and X can represent various substituents among which an unsubstituted C₃-C₇-cycloalkyl. However, there is no explicit disclosure or suggestion to select in this document of any such derivative wherein X can represent a substituted C₃-C₇-cycloalkyl.

In international patent application WO-2008/037789 certain N-cycloalkyl-N-(heteroarylmethyl) (thio)carboxamides are generically embraced in a broad disclosure of numerous compounds of the following formula: 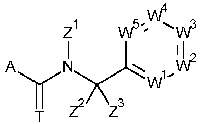 wherein A represents a carbo-linked, partially saturated or unsaturated, 5-membered heterocyclyl group, T can represent O or S, Z¹ represents a substituted or non-substituted C₃-C₇-cycloalkyl group, W¹ to W⁵ can independently represent N or CR^{b} and at least one W¹ to W⁵ represents N, and R^{b} can represent various substituents among which an unsubstituted C₃-C₇-cycloalkyl. However, there is no explicit disclosure or suggestion to select in this document of any such derivative wherein R^{b} can represent a substituted C₃-C₇-cycloalkyl.

In international patent applications WO-2009/016221 and WO-2009/016222 certain N-cycloalkyl-N-(heteroarylmethyl)(thio)carboxamides are generically embraced in a broad disclosure of numerous compounds of the following formula: 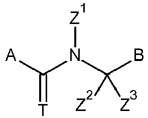 wherein A represents a carbo-linked, partially saturated or unsaturated, 5-membered heterocyclyl group, T can represent O or S, Z¹ represents a substituted or non-substituted C₃-C₇-cycloalkyl group, B can represent a carbo-linked, unsaturated, benzofused, 5- or 6-membered heterocyclyl group that can be substituted by at least a R^{b1}, R^{b2} or R^{b3} group, and R^{b1}, R^{b2} or R^{b3} can represent various substituents among which an unsubstituted C₃-C₇-cycloalkyl. However, there is no explicit disclosure or suggestion to select in this document of any such derivative wherein R^{b1}, R^{b2} or R^{b3} can represent a substituted C₃-C₇-cycloalkyl.
In international patent applications WO-2010/130767 certain N-cycloalkyl-N-(2-pyridylmethyl)(thio) carboxamides are generically embraced in a broad disclosure of numerous compounds of the following formula: 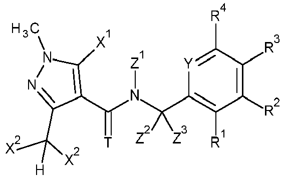 wherein X¹ and X² represent a fluorine of a chlorine atom, T can represent O or S, Z¹ represents a substituted or non-substituted cyclopropyl group or a substituted or non-substituted C₄-C₇-cycloalkyl group, Y can represent N and each substituent Rⁱ, i being an integer from 1 to 4, can, independently, represent various substituents among which a substituted or unsubstituted C₃-C₇-cycloalkyl. However, there is no explicit disclosure or suggestion to select in this document of any such derivative wherein R¹ can represent a substituted C₃-C₇-cycloalkyl when Y represents N.

In international patent application WO-2012/059497 certain N-cycloalkyl-N-(heteroarylmethyl)(thio) carboxamides are generically embraced in a broad disclosure of numerous compounds of the following formula: 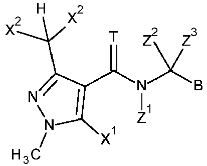 wherein X¹ and X² represent a fluorine of a chlorine atom, T can represent O or S, Z¹ represents a substituted or non-substituted C₃-C₇-cycloalkyl group, B can represent a saturated, partially saturated or unsaturated, monocyclic or fused bicyclic 4-, 5-, 6-, 7-, 8-, 9-, 10-membered ring that can be substituted by at least a X group, and X can represent various substituents among which a substituted or unsubstituted C₃-C₇-cycloalkyl. However, there is no explicit disclosure or suggestion to select in this document of any such derivative wherein X can represent a substituted C₃-C₇-cycloalkyl.

In international patent application WO-2014/172191 certain N-cycloalkyl-N-(heteroarylmethyl)(thio) carboxamides are generically embraced in a broad disclosure of numerous compounds of the following formula: 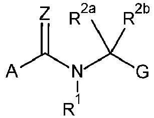 wherein A can represents various carbo-linked, partially saturated or unsaturated, 5- or 6-membered heterocyclyl groups, Z can represent O or S, R¹ can represent a substituted or non-substituted C₃-C₅-cycloalkyl group, G can represent a pyridinyl, pyridazinyl or pyrazinyl group optionally substituted by R³, and R³ can represent various substituents among which a unsubstituted C₃-C₅-cycloalkyl. However, there is no explicit disclosure or suggestion to select in this document of any such derivative wherein R³ can represent a substituted C₃-C₇-cycloalkyl. Accordingly, the present invention provides a N-cycloalkyl-N-{[ortho-(1-substitutedcycloalkyl)heteroaryl]-methylene} (thio)carboxamide of formula (I) 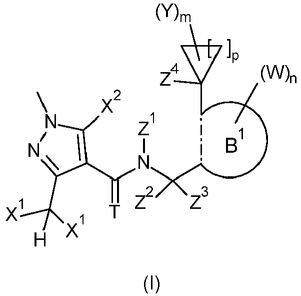 wherein
- X¹ and X² which can be the same or different, represent a chlorine or a fluorine atom ,
- T represents O or S ;
- n represents 0, 1, 2, 3 or 4 ;
- m represents 0, 1, 2, 3, or 4 ;
- p represents 1;
- Z¹ represents a non-substituted cyclopropyl or a cyclopropyl substituted by one or more C₁-C₈-alkyl;
- Z² and Z³, which can be the same or different, represent a hydrogen atom ; or non-substituted C₁-C₈-alkyl ;
- Z⁴ represents a halogen atom; non-substituted C₁-C₈-alkyl ; or non-substituted C₁-C₈-alkoxy; B¹ represents a thienyl ring or a pyridinyl ring ;
- W independently represents a halogen atom or non-substituted C₁-C₈-alkyl ;
- Y independently represents a C₁-C₈-alkyl ;
as well as its salts, N-oxides, metal complexes, metalloid complexes and optically active isomers or geometric isomers thereof.

The following generic terms are generally used with the following meanings:
• halogen means fluorine, chlorine, bromine or iodine ,
   carboxy means -C(=O)OH ;
   carbonyl means -C(=O)- ;
   carbamoyl means -C(=O)NH₂ ;
   N-hydroxycarbamoyl means -C(=O)NHOH ;
   SO represents a sulfoxide group ;
   SO₂ represents a sulfone group ;
   heteroatom means sulfur, nitrogen or oxygen ;
   methylene means the diradical -CH2- ;
• an alkyl group, an alkenyl group and an alkynyl group as well as moieties containing these terms, can be linear or branched ;
• halogenated groups, notably haloalkyl, haloalkoxy and cycloalkyl groups, can comprise up to nine identical or different halogen atoms ;
• the term "aryl" means phenyl or naphthyl ;
• the term "heteroaryl" means a saturated, partially saturated or unsaturated, monocyclic or fused bicyclic 3-, 4-, 5-, 6-, 7-, 8-, 9-, 10-membered ring comprising from 1 up to 4 heteroatoms selected in the list consisting of N, O and S.
• In the case of an amino group or the amino moiety of any other amino-containing group, substituted by two substituents that can be the same or different, the two substituents together with the nitrogen atom to which they are linked can form a heterocyclyl group, preferably a 5- to 7-membered heterocyclyl group, that can be substituted or that can include other hetero atoms, for example a morpholino group or piperidinyl group.
• Where a compound of the invention can be present in tautomeric form, such a compound is understood hereinabove and hereinbelow also to include, where applicable, corresponding tautomeric forms, even when these are not specifically mentioned in each case.

Any of the compounds of the present invention can exist in one or more optical or chiral isomer forms depending on the number of asymmetric centres in the compound. The invention thus relates equally to all the optical isomers and to their racemic or scalemic mixtures (the term "scalemic" denotes a mixture of enantiomers in different proportions) and to the mixtures of all the possible stereoisomers, in all proportions. The diastereoisomers and/or the optical isomers can be separated according to the methods which are known *per se* by the man ordinary skilled in the art.

Any of the compounds of the present invention can also exist in one or more geometric isomer forms depending on the number of double bonds in the compound. The invention thus relates equally to all geometric isomers and to all possible mixtures, in all proportions. The geometric isomers can be separated according to general methods, which are known *per se* by the man ordinary skilled in the art. Any of the compounds of the present invention can also exist in one or more geometric isomer forms depending on the relative position (syn/anti or cis/trans or endo/exo) of the substituents of the chain or ring. The invention thus relates equally to all syn/anti (or cis/trans or endo/exo) isomers and to all possible syn/anti (or cis/trans or endo/exo) mixtures, in all proportions. The syn/anti (or cis/trans or endo/exo) isomers can be separated according to general methods, which are known *per se* by the man ordinary skilled in the art.

Preferred compounds of formula (I) according to the invention are those wherein X¹ represents a fluorine atom.

Other preferred compounds of formula (I) according to the invention are those wherein X² represents a fluorine atom.

Other preferred compounds according to the invention are compounds of formula (I) wherein T represents O.

Other more preferred compounds according to the invention are compounds of formula (I) wherein Z¹ represents a non-substituted cyclopropyl or a cyclopropyl substituted by 1 or 2 C₁-C₅-alkyl;

Other more preferred compounds according to the invention are compounds of formula (I) wherein Z¹ represents a non-substituted cyclopropyl or a 2-C₁-C₅-alkylcyclopropyl.

Other even more preferred compounds according to the invention are compounds of formula (I) wherein Z¹ represents a non-substituted cyclopropyl.

Other even more preferred compounds according to the invention are compounds of formula (I) wherein Z¹ represents a 2-methylcyclopropyl.

Other preferred compounds according to the invention are compounds of formula (I) wherein Z² and Z³ independently represent a hydrogen atom or a methyl.

Other more preferred compounds according to the invention are compounds of formula (I) wherein Z² represents a hydrogen atom and Z³ represents a hydrogen atom or a methyl.

Other more preferred compounds according to the invention are compounds of formula (I) wherein Z² and Z³ represent a hydrogen atom.

Other preferred compounds according to the invention are compounds of formula (I) wherein n represents 0, 1 or 2, even preferred 0 or 1.

Other preferred compounds according to the invention are compounds of formula (I) wherein m represents 0, 1 or 2, even more preferred 0.

Other preferred compounds according to the invention are compounds of formula (I) wherein Z⁴ represents a halogen, non-substituted C₁-C₄-alkyl or non-substituted C₁-C₄-alkyloxy.

Other more preferred compounds according to the invention are compounds of formula (I) wherein Z⁴ represents chloro, methyl or methoxy.

Other preferred compounds according to the invention are compounds of formula (I) wherein W independently, represents a chloro atom, a bromo atom or methyl.

The above mentioned preferences with regard to the substituents of the compounds according to the invention can be combined in various manners. These combinations of preferred features thus provide sub-classes of compounds according to the invention. Examples of such sub-classes of preferred compounds according to the invention are:
- preferred features of X¹ with preferred features of X², T, Z¹ to Z⁴, n, m, B¹, W and Y;
- preferred features of X² with preferred features of X¹, T, Z¹ to Z⁴, n, m, B¹, W and Y;
- preferred features of T with preferred features of X¹, X², Z¹ to Z⁴, n, m, B¹, W and Y;
- preferred features of Z¹ with preferred features of X¹, X², T, Z² to Z⁴, n, m, B¹, W and Y;
- preferred features of Z² with preferred features of X¹, X², T, Z¹, Z³ to Z⁴, n, m, B¹, W and Y;
- preferred features of Z³ with preferred features of X¹, X², T, Z¹ to Z², Z⁴, n, m, B¹, W and Y;
- preferred features of Z⁴ with preferred features of X¹, X², T, Z¹ to Z³, n, m, B¹, W and Y;
- preferred features of n with preferred features of X¹, X², T, Z¹ to Z⁴, m, B¹, W and Y;
- preferred features of m with preferred features of X¹, X², T, Z¹ to Z⁴, n, B¹, W and Y;
- preferred features of B¹ with preferred features of X¹, X², T, Z¹ to Z⁴, n, m, W and Y;
- preferred features of W with preferred features of X¹, X², T, Z¹ to Z⁴, n, m, B¹ and Y;
- preferred features of Y with preferred features of X¹, X², T, Z¹ to Z⁴, n, m, B¹ and W.

In these combinations of preferred features of the substituents of the compounds according to the invention, the said preferred features can also be selected among the more preferred features of each of X¹, X², T, Z¹ to Z⁴, n, m, B¹, W and Y so as to form most preferred subclasses of compounds according to the invention.

In a particular embodiment of the invention, the following preferred features are combined:
- X¹ is a fluorine atom;
- X² is a chlorine or a fluorine atom; particularly a fluorine atom;
- T represents O or S; particularly O;
- Z¹ represents a non-substituted cyclopropyl or a cyclopropyl substituted by 1 or 2 C₁-C₅-alkyl; particularly a non-substituted cyclopropyl or a 2-C₁-C₅-alkylcyclopropyl; even more particularly a a non-substituted cyclopropyl or a 2-methylcyclopropyl;
- Z² and Z³ independently represent a hydrogen atom or a methyl; particularly Z² represents a hydrogen atom and Z³ represents a hydrogen atom or a methyl; even more particularly Z² and Z³ represent a hydrogen atom;
- n represents 0, 1 or 2; particularly 0 or 1 ;
- m represents 0, 1 or 2; even particularly 0;
- Z⁴ represents a halogen, non-substituted C₁-C₄-alkyl or non-substituted C₁-C₄-alkyloxy; even more particularly chloro, methyl or methoxy;
- W independently represents a chloro atom, a bromo atom or methyl;

The present invention also relates to a process for the preparation of the compound of formula (I). Thus, according to a further aspect of the present invention there is provided a process P1 for the preparation of a compound of formula (I) as herein-defined and wherein T represents O and that comprises reaction of an amine of formula (II) or one of its salts: 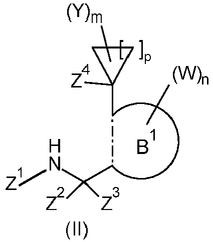 wherein Z¹, Z², Z³, Z⁴, n, m, p, B¹, W and Y are as herein-defined; with a carboxylic acid derivative of formula (III): 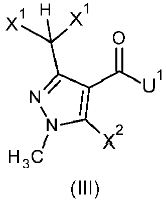 wherein X¹ and X² are as herein-defined and U¹ represents a leaving group selected in the list consisting of a halogen atom, a hydroxyl group, -OR^{a}, -OC(=O)R^{a}, R^{a} being a substituted or non-substituted C₁-C₆-alkyl, a substituted or non-substituted C₁-C₆-haloalkyl, a benzyl, 4-methoxybenzyl or pentafluorophenyl group ; in the presence, if necessary, of a catalyst and in the presence of a condensing agent in case U¹ represents a hydroxyl group, and in the presence of an acid binder in case U¹ represents a halogen atom. N-substituted amine derivatives of formula (II) are known or can be prepared by known processes such as reductive amination of aldehydes of formula (IV): 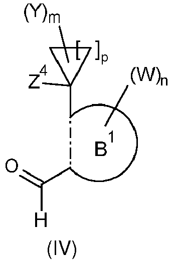 wherein Z⁴, n, m, p, B¹, W and Y are as herein-defined, or reductive amination of ketones (Bioorganics and Medicinal Chemistry Letters (2006), 16, 2014), or reduction of imines (Tetrahedron (2005), 61, 11689), or nucleophilic substitution by an amine of halogenomethylheterocyclic derivatives of formula (Va): 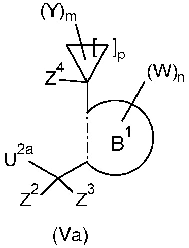 wherein U^{2a} is a halogen, preferentially chloro, bromo and iodo, and Z⁴, n, m, p, B¹, W and Y are as herein-defined, or nucleophilic substitution by an amine of (aryl- or alkyl-sulfonyloxy)methylheterocyclic derivatives of formula (Vb): 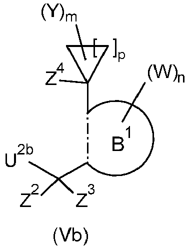 wherein U^{2b} is an arylsulfonate or alkylsulfonate, preferentially tosylate or mesylate, and Z⁴, n, m, p, B¹, W and Y are as herein-defined (Journal of Medicinal Chemistry (2002), 45, 3887).

Compounds of formula (Vb) wherein Z² and Z³ are hydrogen and U^{2b}, Z⁴, n, m, p, B¹, W and Y are as herein-defined can be prepared by sulfonation of a hydroxymethylheterocyclic derivatives of formula (Vc): 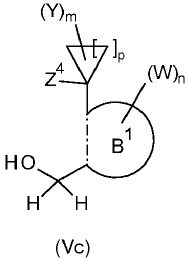 wherein Z⁴, n, m, p, B¹, W and Y are as herein-defined, with a sulfonyl chloride in presence of a base (WO-2014/9495, step 3, page 61).

Carboxylic acid derivatives of formula (III) can be prepared according to WO-2010/130767.

In case U¹ represents a hydroxy group, process P1 according to the present invention is conducted in the presence of condensing agent. Suitable condensing agent may be selected in the non limited list consisting of acid halide former, such as phosgene, phosphorous tribromide, phosphorous trichloride, phosphorous pentachloride, phosphorous trichloride oxide or thionyl chloride; anhydride former, such as ethyl chloroformate, methyl chloroformate, isopropyl chloroformate, isobutyl chloroformate or methanesulfonyl chloride; carbodiimides, such as N,N'-dicyclohexylcarbodiimide (DCC) or other customary condensing agents, such as phosphorous pentoxide, polyphosphoric acid, N,N'-carbonyldiimidazole, 2-ethoxy-N-ethoxycarbonyl-1,2-dihydroquinoline (EEDQ), triphenylphosphine/tetrachloromethane, 4-(4,6-dimethoxy[1.3.5]-triazin-2-yl)-4-methylmorpholinium chloride hydrate, bromo-tripyrrolidinophosphoniumhexafluorophosphate or propanephosphonic anhydride (T3P).
Process P1 according to the present invention may be conducted in the presence of a catalyst. Suitable catalyst may be selected in the list consisting of N,N-dimethylpyridin-4-amine, 1-hydroxy-benzotriazole or N,N-dimethylformamide.

In case U¹ represents a halogen atom, process P1 according to the present invention is conducted in the presence of an acid binder. Suitable acid binders for carrying out process P1 according to the invention are in each case all inorganic and organic bases that are customary for such reactions. Preference is given to using alkaline earth metal, alkali metal hydride, alkali metal hydroxides or alkali metal alkoxides, such as sodium hydroxide, sodium hydride, calcium hydroxide, potassium hydroxide, potassium tert-butoxide or other ammonium hydroxide, alkali metal carbonates, such as caesium carbonate, sodium carbonate, potassium carbonate, potassium bicarbonate, sodium bicarbonate, alkali metal or alkaline earth metal acetates, such as sodium acetate, potassium acetate, calcium acetate and also tertiary amines, such as trimethylamine, triethylamine, diisopropylethylamine, tributylamine, N,N-dimethylaniline, pyridine, N-methylpiperidine, N,N-dimethylpyridin-4-amine, diazabicyclooctane (DABCO), diazabicyclononene (DBN) or diazabicycloundecene (DBU).

It is also possible to work in the absence of an additional condensing agent or to employ an excess of the amine component, so that it simultaneously acts as acid binder agent.

Suitable solvents for carrying out process P1 according to the invention can be customary inert organic solvents. Preference is given to using optionally halogenated aliphatic, alicyclic or aromatic hydrocarbons, such as petroleum ether, hexane, heptane, cyclohexane, methylcyclohexane, benzene, toluene, xylene or decalin; chlorobenzene, dichlorobenzene, dichloromethane, chloroform, carbon tetrachloride, dichlorethane or trichlorethane; ethers, such as diethyl ether, diisopropyl ether, methyl t-butyl ether, methyl t-amyl ether, dioxane, tetrahydrofuran, 1,2-dimethoxyethane, 1,2-diethoxyethane or anisole; nitriles, such as acetonitrile, propionitrile, n- or i-butyronitrile or benzonitrile; amides, such as N,N-dimethylformamide, N,N-dimethylacetamide, N-methylformanilide, N-methylpyrrolidone, or hexamethylphosphoric triamide; alcohols such as methanol, ethanol, propanol, iso-propanol; esters, such as methyl acetate or ethyl acetate, sulfoxides, such as dimethyl sulfoxide, or sulfones, such as sulfolane.

When carrying out process P1 according to the invention, the amine derivative of formula (II) can be employed as its salt, such as chlorhydrate or any other convenient salt.

When carrying out process P1 according to the invention, 1 mole or an excess of the amine derivative of formula (II) and from 1 to 3 moles of the acid binder can be employed per mole of the reagent of formula (III).

It is also possible to employ the reaction components in other ratios. Work-up is carried out by known methods.

According to a further aspect according to the invention, there is provided a second process P2 for the preparation of a compound of formula (I) wherein T represents S, starting from a compound of formula (I) wherein T represents O and illustrated according to the following reaction scheme : 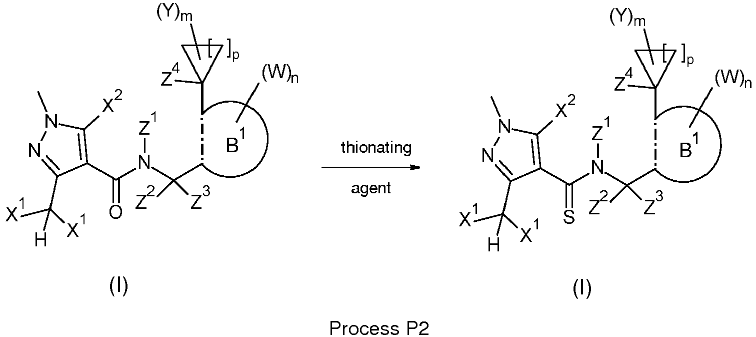 wherein X¹, X², Z¹, Z², Z³, Z⁴, n, m, p, B¹, W and Y are as herein-defined.

Process P2 according to the invention is performed in the presence of a thionating agent.

Starting amide derivatives of formula (I) wherein T represents O can be prepared according to process P1.

Suitable thionating agents for carrying out process P2 according to the invention can be sulfur (S), sulfhydric acid (H₂S), sodium sulfide (Na₂S), sodium hydrosulfide (NaHS), boron trisulfide (B₂S₃), bis(diethylaluminium) sulfide ((AlEt₂)₂S), ammonium sulfide ((NH₄)₂S), phosphorous pentasulfide (P2S5), Lawesson's reagent (2,4-bis(4-methoxyphenyl)-1,2,3,4-dithiadiphosphetane 2,4-disulfide) or a polymer-supported thionating reagent such as described in Journal of the Chemical Society, Perkin 1 (2001), 358, in the optionally presence of a catalytic or stoichiometric or excess amount, quantity of a base such as an inorganic and organic base. Preference is given to using alkali metal carbonates, such as sodium carbonate, potassium carbonate, potassium bicarbonate, sodium bicarbonate ; heterocyclic aromatic bases, such as pyridine, picoline, lutidine, collidine ; and also tertiary amines, such as trimethylamine, triethylamine, tributylamine, N,N-dimethylaniline, N,N-dimethylpyridin-4-amine or N-methyl-piperidine. Suitable solvents for carrying out process P2 according to the invention can be customary inert organic solvents. Preference is given to using optionally halogenated aliphatic, alicyclic or aromatic hydrocarbons, such as petroleum ether, hexane, heptane, cyclohexane, methylcyclohexane, benzene, toluene, xylene or decalin, chlorobenzene, dichlorobenzene, dichloromethane, chloroform, carbon tetrachloride, dichlorethane or trichlorethane, ethers, such as diethyl ether, diisopropyl ether, methyl t-butyl ether, methyl t-amyl ether, dioxane, tetrahydrofuran, 1,2-dimethoxyethane or 1,2-diethoxyethane, nitriles, such as acetonitrile, propionitrile, n- or i-butyronitrile or benzonitrile, sulfurous solvents, such as sulfolane or carbon disulfide.

When carrying out process P2 according to the invention, 1 mole or an excess of the sulfur equivalent of the thionating agent and from 1 to 3 moles of the base can be employed per mole of the amide reactant (I).
It is also possible to employ the reaction components in other ratios. Work-up is carried out by known methods.

When carrying out processes P1 and P2 according to the invention, the reaction temperatures can be varied within a relatively wide range. In general, these processes are carried out at temperatures from 0 °C to 200 °C, preferably from 10 °C to 150 °C. A way to control the temperature for the processes according to the invention is to use microwave technology.

The present invention also relates to a process for the preparation of the compound of formula (IV) (Process P3) : 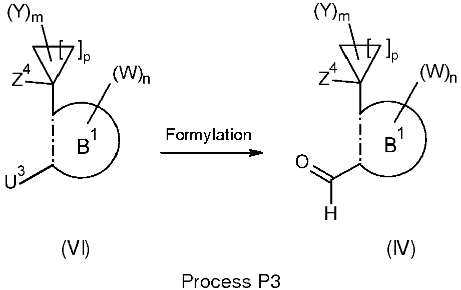 wherein U³ is defined as bromo or iodo, and Z⁴, n, m, p, B¹, W and Y are as herein-defined.

A compound of the general formula (IV) is obtained from a compound of the general formula (VI) by a formylation reaction, such as a reaction sequence of halogen-metal exchange with an organolithium or an organomagnesium reagent, followed by subsequent addition of an electrophile (e.g. N,N-dimethylformamide (DMF)) (Journal of the American Chemical Society, (2008), 130, 8481-8490). Alternatively, magnesium can be used to form a Grignard reagent from (VI), which can then be treated with an appropriate electrophile such as DMF to form a compound of the general formula (IV) (Chemistry Letters, (2007), 36, 72-73).

Process P3 is performed in the presence of a suitable organometallic compound or magnesium. Preferred organometallic compounds are organolithium compounds (such as butyllithium) or organomagnesium compounds (such us *iso*-propylmagnesium chloride or bromide).
Process P3 is preferably performed using one or more diluents. Useful solvents in the performance of process P3 are preferably aprotic solvents (such as dioxane, glyme, alkanes, cycloalkanes, diethyl ether or tetrahydrofuran). Particular preference is given to diethyl ether or tetrahydropyran.
In the performance of process P3, the reaction temperatures can be varied within a relatively wide range. In the case of the halogen-metal exchange reactions, the temperatures employed are generally from -120 °C to 150 °C, preferably temperatures from -12 0 °C to 60 °C, most preferably -120 °C to 70 °C. Af ter the addition of an electrophile such as DMF, preference is given to working at -80 °C to 50 °C.
To perform process P3, generally 1 to 2 mol, preferably 1 mol, of the organometallic compound and of the electrophile are used per mole of compound of the formula (VI).

The present invention also relates to processes for the preparation of the compound of formula (Va1) (Process P4) : 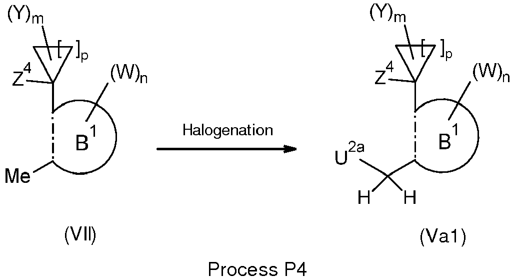 wherein U^{2a} is a halogen, preferentially chloro, bromo and iodo, and Z⁴, n, m, p, B¹, W and Y are as herein-defined.

A compound of the general formula (Va1) is obtained from a compound of the general formula (VII) by a radical halogenation of the methyl group (WO-2008/016239 and WO-2013/051632).

Process P4 is performed in the presence of a suitable halogenating reagent (such as n-chlorosuccinimide, n-bromosuccinimide, chlorine, bromine, iodine), and with a catalytic amount of a radical initiator such as 2,2'-Azobis(2-methylpropionitrile (AIBN).
Process P4 is preferably performed using one or more diluents. Useful solvents in the performance of process P4 are preferably inert solvents under radical halogenation conditions (such as carbon tetrachloride). Particular preference is given to carbon tetrachloride.
In the performance of process P4, the reaction temperatures can be varied within a relatively wide range. The temperatures employed are generally from -120 °C to 200 °C, preferably temperatures from 80 °C to 150 °C.
To perform process P4, 1 to 2 mol, preferably 1 mol, of a halogenating reagent are generally used per mole of compound of the formula (VII).

The present invention also relates to processes for the preparation of the compound of formula (VI) and (VII) wherein Z⁴ is an electron withdrawing group (Process P5) : 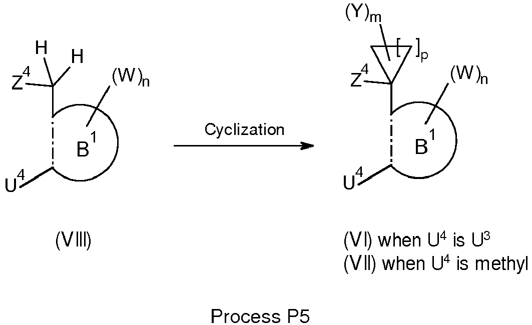 wherein U⁴ is defined as methyl, chloro, bromo or iodo, and Z⁴, n, m, p, B¹, W and Y are as herein-defined.

A compound of the general formula (VI) or (VII) is obtained from a compound of the general formula (VIII), wherein Z⁴ is an electron withdrawing group (such as nitrile, carboxylic acid ester), by a cyclization reaction with a substituted or non-substituted alkyl chain which bears an appropriate leaving group, such as chloro, bromo, iodo, mesylate, tosylate or triflate, on each terminal carbon (such as 1,2-dibromoethane, 1,4-dibromobutane or 1,5-dibromopentane), in the presence of a suitable base, as mineral carbonates, such as potassium carbonate, sodium carbonate or caesium carbonate; metal hydroxides such as sodium hydroxide or potassium hydroxide; alkoxides, such as potassium *tert*-butoxide or sodium *tert*-butoxide; metal hydrides, such as sodium hydride; amides, such as lithium diisopropylamide (Organic & Biomolecular Chemistry, (2012), 10, 6404-6409 and WO-2011/041694). Process P5 can also be performed in the presence of an additive such as tetra-n-butylammonium bromide or N,N'-Dimethyl-N,N'-trimethyleneurea (DMPU).
To perform process P5, generally a stoichiometric or an excess amount of the substituted or non-substituted alkyl chain which bears an appropriate leaving group on each terminal carbon is used per mole of compound of the formula (VIII).
The solvents used may be all customary solvents which are inert under the reaction conditions, or the reaction can be performed in mixtures of two or more of these solvents.
In the performance of process P5, the reaction temperatures can be varied within a relatively wide range. In general, the temperatures employed are from -10 °C to 150 °C, preferably temperatures from 0 °C to 100 °C.

The present invention also relates to processes for the preparation of the compound of formula (VI) and (VII) wherein p is 1 (Process P6) : 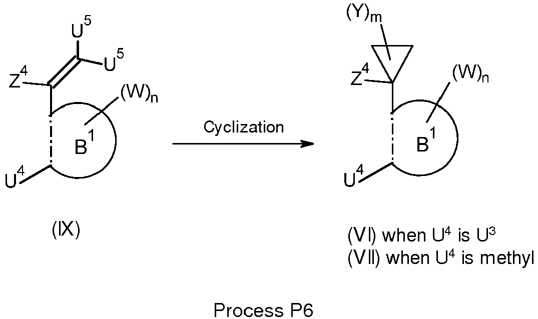 wherein U⁴ is defined as methyl, chloro, bromo or iodo, U⁵ is defined as hydrogen or Y , and Z⁴, n, m, B¹, W and Y are as herein-defined.

A compound of the general formula (VI) or (VII) wherein p is 1, is obtained from a compound of the general formula (IX) by a cyclopropanation reaction such as the Simmons-Smith reaction (WO-2012/165648) ; cyclopropanation with a free carbene (Chemical Reviews, (2003), 103, 1099-1132) ; or cyclopropanation with a metal carbinoid (Chemical Reviews, (1987), 87, 411-432).
Alkenes derivatives (IX) are commercially available or can be prepared from commercially available precursors by methods described in the literature, from ketones by a Wittig or Horner-Wadsworth-Emmons olefination (Chemical Reviews, (1989), 89, 863-927) ; a Julia olefination (Tetrahedron Letters, (1973), 14, 4833-4836) ; a Peterson olefination (Journal of Organic Chemistry, (1968), 33, 780) ; or trapping of an electrophile by an enolate or an enol (WO-1991/11445).
The solvents used may be all customary solvents which are inert under the reaction conditions, or the reaction can be performed in mixtures of two or more of these solvents.
In the performance of process P6, the reaction temperatures can be varied within a relatively wide range. In general, the temperatures employed are from -120 °C to 150 °C, preferably temperatures from 80 °C t o 100 °C.

It is recognized that at any appropriate stage of synthesis, the substituent Z⁴ can be converted from one substituent definition to another as specified above, in one or more steps, by synthetic methods commonly used by the person skilled in the art of chemical synthesis, for example, from a nitrile to a corresponding carboxylic acid by hydrolysis or to an aldehyde by reduction ; from a carboxylic acid to a hydroxyalkyl by reduction ; to a halogen by decarboxylative halogenation ; from an aldehyde to a corresponding alkene or alkyne by a Wittig olefination or a Seyferth-Gilbert homologation.

Furthermore, it is also recognized that some reagents and reaction conditions described above for preparation of compounds of the formula (I) may not be compatible with particular functionalities present in the intermediate compounds. In these cases, the introduction of protection/deprotection sequences or of mutual conversions of functional groups into the synthesis helps to obtain the desired products. The use and selection of the protecting groups is obvious to the person skilled in the art of chemical synthesis (see, for example, "Protective Groups in Organic Synthesis"; Third Edition; 494-653, and literature cited therein). The person skilled in the art will recognize that, in some cases, after the introduction of a given reagent as shown in an individual scheme, it may be necessary to perform additional routine synthesis steps not described individually in order to complete the synthesis of compounds of the formula (I). The person skilled in the art will likewise recognize that it may be necessary to perform a combination of the steps illustrated in the above schemes in a sequence other than the implied sequence shown specifically, in order to prepare the compounds of the formula (I).
Processes P1 and P6 according to the invention are generally carried out under atmospheric pressure. It is also possible to operate under elevated or reduced pressure.
In general, the reaction mixture is concentrated under reduced pressure. The residue that remains can be freed by known methods, such as chromatography or crystallization, from any impurities that can still be present.
Work-up is carried out by customary methods. Generally, the reaction mixture is treated with water and the organic phase is separated off and, after drying, concentrated under reduced pressure. If appropriate, the remaining residue can, be freed by customary methods, such as chromatography, crystallization or distillation, from any impurities that may still be present.
The compound according to the present invention can be prepared according to the general processes of preparation described above. It will nevertheless be understood that, on the basis of his general knowledge and of available publications, the skilled worker will be able to adapt this method according to the specifics of each of the compounds, which it is desired to synthesize.
The present invention thus provides compounds of formula (IIa) as well as their acceptable salts: 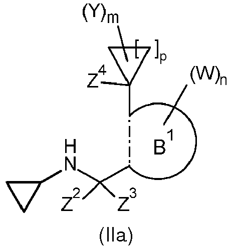 wherein Z², Z³, Z⁴, n, m, p, B¹, W and Y are as herein-defined.

The following compound of formula (IIa) is mentioned in chemical databases and/or suppliers' databases but without any references or information which enables it to be prepared and separated : - N-{[4-(1-methylcyclopropyl)-3-thienyl]methyl}cyclopropanamine [1601214-96-3]

Preferred compounds of formula (IIa) according to the invention are :
- N-{[3-(1-methylcyclopropyl)-2-thienyl]methyl}cyclopropanamine
- N-{[3-(1-chlorocyclopropyl)-2-thienyl]methyl}cyclopropanamine
- N-{[3-(1-chlorocyclopropyl)-5-methyl-2-thienyl]methyl}cyclopropanamine, and
- N-{[3-(1-methoxycyclopropyl)-5-methyl-2-thienyl]methyl}cyclopropanamine. as well as their acceptable salts.

Other interesting compounds of formula (IIa) according to the invention are :
- N-{[2-(1-methylcyclopropyl)pyridin-3-yl]methyl}cyclopropanamine
- N-{[5-methyl-2-(1-methylcyclopropyl)pyridin-3-yl]methyl}cyclopropanamine
- N-{[5-chloro-2-(1-methylcyclopropyl)pyridin-3-yl]methyl}cyclopropanamine, and
- N-{[5-bromo-2-(1-methylcyclopropyl)pyridin-3-yl]methyl}cyclopropanamine. as well as their acceptable salts.

The present invention thus also provides compounds of formula (IV) : 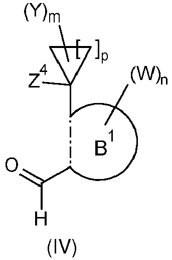 wherein Z⁴, n, m, p, B¹, W and Y are as herein-defined.

Preferred compounds of formula (IV) according to the invention are : - 3-(1-chlorocyclopropyl)thiophene-2-carbaldehyde, and - 3-(1-chlorocyclopropyl)-5-methylthiophene-2-carbaldehyde.

Other interesting compounds of formula (IV) according to the invention are : - 2-(1-methylcyclopropyl)nicotinaldehyde - 5-methyl-2-(1-methylcyclopropyl)nicotinaldehyde - 5-chloro-2-(1-methylcyclopropyl)nicotinaldehyde, and - 5-bromo-2-(1-methylcyclopropyl)nicotinaldehyde.

The present invention thus also provides compounds of formula (Va1) : 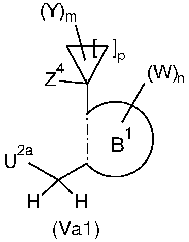 wherein U^{2a} is halogen, and Z⁴, n, m, p, B¹, W and Y are as herein-defined.

Preferred compounds of formula (Va1) according to the invention are :
- 2-(bromomethyl)-3-(1-methylcyclopropyl)thiophene, and
- 2-(bromomethyl)-5-methyl-3-(1-methylcyclopropyl)thiophene.

The present invention thus also provides compounds of formula (Vc) : 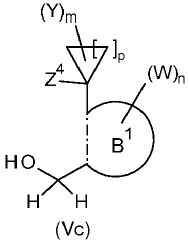 wherein Z⁴, n, m, p, B¹, W and Y are as herein-defined.

Preferred compounds of formula (Vc) according to the invention are :
- [3-(1-methylcyclopropyl)-2-thienyl]methanol
- [5-methyl-3-(1-methylcyclopropyl)-2-thienyl]methanol.

In a further aspect, the present invention also relates to a fungicide composition comprising an effective and non-phytotoxic amount of an active compound of formula (I).

The expression "effective and non-phytotoxic amount" means an amount of composition according to the invention that is sufficient to control or destroy the fungi present or liable to appear on the crops and that does not entail any appreciable symptom of phytotoxicity for the said crops. Such an amount can vary within a wide range depending on the fungus to be controlled, the type of crop, the climatic conditions and the compounds included in the fungicide composition according to the invention. This amount can be determined by systematic field trials that are within the capabilities of a person skilled in the art.

Thus, according to the invention, there is provided a fungicide composition comprising, as an active ingredient, an effective amount of a compound of formula (I) as herein defined and an agriculturally acceptable support, carrier or filler.

According to the invention, the term "support" denotes a natural or synthetic, organic or inorganic compound with that the active compound of formula (I) is combined or associated to make it easier to apply, notably to the parts of the plant. This support is thus generally inert and should be agriculturally acceptable. The support can be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports can also be used.

The composition according to the invention can also comprise additional components. In particular, the composition can further comprise a surfactant. The surfactant can be an emulsifier, a dispersing agent or a wetting agent of ionic or non-ionic type or a mixture of such surfactants. Mention can be made, for example, of polyacrylic acid salts, lignosulfonic acid salts, phenolsulfonic or naphthalenesulfonic acid salts, polycondensates of ethylene oxide with fatty alcohols or with fatty acids or with fatty amines, substituted phenols (in particular alkylphenols or arylphenols), salts of sulfosuccinic acid esters, taurine derivatives (in particular alkyl taurates), phosphoric esters of polyoxyethylated alcohols or phenols, fatty acid esters of polyolsand derivatives of the above compounds containing sulfate, sulfonate and phosphate functions. The presence of at least one surfactant is generally essential when the active compound and/or the inert support are water-insoluble and when the vector agent for the application is water. Preferably, surfactant content can be comprised from 5% to 40% by weight of the composition.

Optionally, additional components can also be included, e.g. protective colloids, adhesives, thickeners, thixotropic agents, penetration agents, stabilisers, sequestering agents. More generally, the active compounds can be combined with any solid or liquid additive, that complies with the usual formulation techniques.

In general, the composition according to the invention can contain from 0.05 to 99% by weight of active compound, preferably 10 to 70% by weight.

Compositions according to the invention can be used in various forms and formulations such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure),gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder. These compositions include not only compositions that are ready to be applied to the plant or seed to be treated by means of a suitable device, such as a spraying or dusting device, but also concentrated commercial compositions that must be diluted before application to the crop.

The formulations can be prepared in a manner known per se, for example by mixing the active ingredients with at least one customary extender, solvent or diluent, adjuvant, emulsifier, dispersant, and/or binder or fixative, wetting agent, water repellent, if appropriate desiccants and UV stabilizers and, if appropriate, dyes and pigments, antifoams, preservatives, inorganic and organic thickeners, adhesives, gibberellins and also further processing auxiliaries and also water. Depending on the formulation type to be prepared further processing steps are necessary, e.g. wet grinding, dry grinding and granulation.
The inventive active ingredients may be present as such or in their (commercial) formulations and in the use forms prepared from these formulations as a mixture with other (known) active ingredients, such as insecticides, attractants, sterilants, bactericides, acaricides, nematicides, fungicides, growth regulators, herbicides, fertilizers, safeners biologicals, and/or semiochemicals.

The compounds of formula (I) and the fungicide composition according to the invention can be used to curatively or preventively control the phytopathogenic fungi of plants or crops, particularly rust diseases.
Thus, according to a further aspect of the invention, there is provided a method for curatively or preventively controlling the phytopathogenic fungi of plants or crops, particularly rust diseases, characterised in that a compound of formula (I) or a fungicide composition according to the invention is applied to the seed, the plant or to the fruit of the plant or to the soil wherein the plant is growing or wherein it is desired to grow.
The method of treatment according to the invention can also be useful to treat propagation material such as tubers or rhizomes, but also seeds, seedlings or seedlings pricking out and plants or plants pricking out. This method of treatment can also be useful to treat roots. The method of treatment according to the invention can also be useful to treat the overground parts of the plant such as trunks, stems or stalks, leaves, flowers and fruit of the concerned plant.

According to the invention all plants and plant parts can be treated. By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms, fruiting bodies, fruits and seed as well as roots, corms and rhizomes are listed. Crops and vegetative and generative propagating material, for example cuttings, corms, rhizomes, runners and seeds also belong to plant parts.
Among the plants that can be protected by the method according to the invention, mention may be made of major field crops like corn, soybean, cotton, *Brassica* oilseeds such as *Brassica napus* (e.g. canola), *Brassica rapa, B. juncea* (e.g. mustard) and *Brassica carinata,* rice, wheat, sugarbeet, sugarcane, oats, rye, barley, millet, triticale, flax, vine and various fruits and vegetables of various botanical taxa such as *Rosaceae sp.* (for instance pip fruit such as apples and pears, but also stone fruit such as apricots, cherries, almonds and peaches, berry fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae* sp. (for instance banana trees and plantings), *Rubiaceae sp.* (for instance coffee), *Theaceae sp., Sterculiceae sp., Rutaceae sp.* (for instance lemons, oranges and grapefruit) ; *Solanaceae sp.* (for instance tomatoes, potatoes, peppers, eggplant), *Liliaceae sp.,* Compositiae sp. (for instance lettuce, artichoke and chicory - including root chicory, endive or common chicory), *Umbelliferae sp.* (for instance carrot, parsley, celery and celeriac), *Cucurbitaceae sp.* (for instance cucumber - including pickling cucumber, squash, watermelon, gourds and melons), *Alliaceae* sp. (for instance onions and leek), Cruciferae sp. (for instance white cabbage, red cabbage, broccoli, cauliflower, brussel sprouts, pak choi, kohlrabi, radish, horseradish, cress, Chinese cabbage), Leguminosae sp. (for instance peanuts, peas and beans beans - such as climbing beans and broad beans), *Chenopodiaceae sp.* (for instance mangold, spinach beet, spinach, beetroots), *Malvaceae* (for instance okra), *Asparagaceae* (for instance asparagus); horticultural and forest crops; ornamental plants; as well as genetically modified homologues of these crops.

Wild plant species and plant cultivars, or those obtained by conventional biological breeding methods, such as crossing or protoplast fusion, and parts thereof, can be treated by the above disclosed methods.Transgenic plants and plant cultivars obtained by genetic engineering methods, if appropriate in combination with conventional methods (Genetically Modified Organisms), and parts thereof can be treated by the above disclosed methods. Preferably, plants of the plant cultivars which are commercially available or are in use are treated in accordance with the invention. Plant cultivars are understood to mean plants which have new properties ("traits") and have been obtained by conventional breeding, by mutagenesis or by recombinant DNA techniques. They can be cultivars, varieties, bio- or genotypes.

The disclosed methods can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Plants and plant cultivars which can be treated by the above disclosed methods include all plants which have genetic material which impart particularly advantageous, useful traits to these plants (whether obtained by breeding and/or biotechnological means).

Plants and plant cultivars which can be treated by the above disclosed methods include plants and plant cultivars which are resistant against one or more biotic stresses, i.e. said plants show a better defense against animal and microbial pests, such as against nematodes, insects, mites, phytopathogenic fungi, bacteria, viruses and/or viroids.

Plants and plant cultivars which can be treated by the above disclosed methods include those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which can be treated by the above disclosed methods include those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content and composition for example cotton or starch, protein content, oil content and composition, nutritional value, reduction in anti-nutritional compounds, improved processability and better storage stability.

Plants and plant cultivars which can be treated by the above disclosed methods include plants and plant cultivars which are hybrid plants that already express the characteristic of heterosis or hybrid vigor which results in generally higher yield, vigor, health and resistance towards biotic and abiotic stresses).

Plants and plant cultivars (obtained by plant biotechnology methods such as genetic engineering) which can be treated by the above disclosed methods include plants and plant cultivars which are herbicide-tolerant plants, i.e. plants made tolerant to one or more given herbicides. Such plants can be obtained either by genetic transformation, or by selection of plants containing a mutation imparting such herbicide tolerance.

Plants and plant cultivars (obtained by plant biotechnology methods such as genetic engineering) which can be treated by the above disclosed methods include plants and plant cultivars which are insect-resistant transgenic plants, i.e. plants made resistant to attack by certain target insects. Such plants can be obtained by genetic transformation, or by selection of plants containing a mutation imparting such insect resistance.

Plants and plant cultivars (obtained by plant biotechnology methods such as genetic engineering) which can be treated by the above disclosed methods include plants and plant cultivars which are tolerant to abiotic stresses. Such plants can be obtained by genetic transformation, or by selection of plants containing a mutation imparting such stress resistance.

Plants and plant cultivars (obtained by plant biotechnology methods such as genetic engineering) which can be treated by the above disclosed methods include plants and plant cultivars which show altered quantity, quality and/or storage-stability of the harvested product and/or altered properties of specific ingredients of the harvested product.

Plants and plant cultivars (obtained by plant biotechnology methods such as genetic engineering) which can be treated by the above disclosed methods include plants and plant cultivars, such as cotton plants, with altered fiber characteristics. Such plants can be obtained by genetic transformation, or by selection of plants contain a mutation imparting such altered fiber characteristics.

Plants and plant cultivars (obtained by plant biotechnology methods such as genetic engineering) which can be treated by the above disclosed methods include plants and plant cultivars, such as oilseed rape or related Brassica plants, with altered oil profile characteristics. Such plants can be obtained by genetic transformation, or by selection of plants contain a mutation imparting such altered oil profile characteristics.

Plants and plant cultivars (obtained by plant biotechnology methods such as genetic engineering) which can be treated by the above disclosed methods include plants and plant cultivars, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering.

Plants and plant cultivars (obtained by plant biotechnology methods such as genetic engineering) which can be treated by the above disclosed methods include plants and plant cultivars, such as Tobacco plants, with altered post-translational protein modification patterns.

Among the diseases of plants or crops that can be controlled by the method according to the invention, mention can be made of :
Powdery mildew diseases such as :
   Blumeria diseases, caused for example by *Blumeria graminis ;*
   Podosphaera diseases, caused for example by *Podosphaera leucotricha ;*
   Sphaerotheca diseases, caused for example by *Sphaerotheca fuliginea ;*
   Uncinula diseases, caused for example by *Uncinula necator;*
Rust diseases such as :
   Gymnosporangium diseases, caused for example by *Gymnosporangium sabinae ;*
   Hemileia diseases, caused for example by *Hemileia vastatrix;*
   Phakopsora diseases, caused for example by *Phakopsora pachyrhizi* or *Phakopsora meibomiae ;*
   Puccinia diseases, caused for example by *Puccinia recondite, Puccinia graminis or Puccinia striiformis*;
   Uromyces diseases, caused for example by *Uromyces appendiculatus*;
Oomycete diseases such as :
   Albugo diseases caused for example by *Albugo candida;*
   Bremia diseases, caused for example by *Bremia lactucae ;*
   Peronospora diseases, caused for example by *Peronospora pisi* or P. *brassicae ;*
   Phytophthora diseases, caused for example by *Phytophthora infestans*;
   Plasmopara diseases, caused for example by *Plasmopara viticola ;*
   Pseudoperonospora diseases, caused for example by *Pseudoperonospora humuli* or *Pseudoperonospora cubensis ;*
   Pythium diseases, caused for example by *Pythium ultimum ;*
Leafspot, leaf blotch and leaf blight diseases such as :
   Alternaria diseases, caused for example by *Alternaria solani;*
   Cercospora diseases, caused for example by *Cercospora beticola ;*
   Cladiosporum diseases, caused for example by *Cladiosporium cucumerinum*;
   Cochliobolus diseases, caused for example by *Cochliobolus sativus* (Conidiaform: Drechslera, Syn: Helminthosporium) or *Cochliobolus miyabeanus ;*
   Colletotrichum diseases, caused for example by *Colletotrichum lindemuthanium*;
   Cycloconium diseases, caused for example by *Cycloconium oleaginum*;
   Diaporthe diseases, caused for example by *Diaporthe citri;*
   Elsinoe diseases, caused for example by *Elsinoe fawcettii;*
   Gloeosporium diseases, caused for example by *Gloeosporium laeticolor;*
   Glomerella diseases, caused for example by *Glomerella cingulata ;*
   Guignardia diseases, caused for example by *Guignardia bidwelli*;
   Leptosphaeria diseases, caused for example by *Leptosphaeria maculans*; *Leptosphaeria nodorum ;*
   Magnaporthe diseases, caused for example by *Magnaporthe grisea ;*
   Mycosphaerella diseases, caused for example by *Mycosphaerella graminicola* ; Mycosphaerella arachidicola ; *Mycosphaerella fijiensis ;*
   Phaeosphaeria diseases, caused for example by *Phaeosphaeria nodorum ;*
   Pyrenophora diseases, caused for example by *Pyrenophora teres,* or *Pyrenophora tritici repentis ;*
   Ramularia diseases, caused for example by *Ramularia collocygni,* or *Ramularia areola* ;
   Rhynchosporium diseases, caused for example by *Rhynchosporium secalis ;*
   Septoria diseases, caused for example by *Septoria apii* or *Septoria lycopercisi*;
   Typhula diseases, caused for example by *Typhula incarnata* ;
   Venturia diseases, caused for example by *Venturia inaequalis ;*
Root, Sheath and stem diseases such as :
   Corticium diseases, caused for example by *Corticium graminearum ;*
   Fusarium diseases, caused for example by *Fusarium oxysporum ;*
   Gaeumannomyces diseases, caused for example by *Gaeumannomyces graminis ;*
   Rhizoctonia diseases, caused for example by *Rhizoctonia solani*;
   Sarocladium diseases caused for example by *Sarocladium oryzae;*
   Sclerotium diseases caused for example by *Sclerotium oryzae;*
   Tapesia diseases, caused for example by *Tapesia acuformis ;*
   Thielaviopsis diseases, caused for example by *Thielaviopsis basicola ;*
Ear and panicle diseases such as :
   Alternaria diseases, caused for example by *Alternaria spp. ;*
   Aspergillus diseases, caused for example by *Aspergillus flavus*;
   Cladosporium diseases, caused for example by *Cladosporium spp. ;*
   Claviceps diseases, caused for example by *Claviceps purpurea ;*
   Fusarium diseases, caused for example by *Fusarium culmorum* ;
   Gibberella diseases, caused for example by *Gibberella zeae ;*
   Monographella diseases, caused for example by *Monographella nivalis ;*
Smut and bunt diseases such as :
   Sphacelotheca diseases, caused for example by *Sphacelotheca reiliana ;*
   Tilletia diseases, caused for example by *Tilletia caries ;*
   Urocystis diseases, caused for example by *Urocystis occulta ;*
   Ustilago diseases, caused for example by *Ustilago nuda ;*
Fruit rot and mould diseases such as :
   Aspergillus diseases, caused for example by *Aspergillus flavus* ;
   Botrytis diseases, caused for example by *Botrytis cinerea ;*
   Penicillium diseases, caused for example by *Penicillium expansum* ;
   Rhizopus diseases caused by example by *Rhizopus stolonifer*
   Sclerotinia diseases, caused for example by *Sclerotinia sclerotiorum ;*
   Verticilium diseases, caused for example by *Verticilium alboatrum ;*
Seed and soilborne decay, mould, wilt, rot and damping-off diseases :
   Alternaria diseases, caused for example by *Alternaria brassicicola ;*
   Aphanomyces diseases, caused for example by *Aphanomyces euteiches ;*
   Ascochyta diseases, caused for example by *Ascochyta lentis ;*
   Aspergillus diseases, caused for example by *Aspergillus flavus ;*
   Cladosporium diseases, caused for example by *Cladosporium herbarum ;*
   Cochliobolus diseases, caused for example by *Cochliobolus sativus ;*
   (Conidiaform: *Drechslera, Bipolaris Syn: Helminthosporium) ;*
   Colletotrichum diseases, caused for example by *Colletotrichum coccodes ;*
   Fusarium diseases, caused for example by *Fusarium culmorum ;*
   Gibberella diseases, caused for example by *Gibberella zeae ;*
   Macrophomina diseases, caused for example by *Macrophomina phaseolina ;*
   Monographella diseases, caused for example by *Monographella nivalis ;*
   Penicillium diseases, caused for example by *Penicillium expansum ;*
   Phoma diseases, caused for example by *Phoma lingam ;*
   Phomopsis diseases, caused for example by *Phomopsis sojae ;*
   Phytophthora diseases, caused for example by *Phytophthora cactorum ;*
   Pyrenophora diseases, caused for example by *Pyrenophora graminea ;*
   Pyricularia diseases, caused for example by *Pyricularia oryzae ;*
   Pythium diseases, caused for example by *Pythium ultimum ;*
   Rhizoctonia diseases, caused for example by *Rhizoctonia solani* ;
   Rhizopus diseases, caused for example by *Rhizopus oryzae ;*
   Sclerotium diseases, caused for example by *Sclerotium rolfsii ;*
   Septoria diseases, caused for example by *Septoria nodorum ;*
   Typhula diseases, caused for example by *Typhula incarnata ;*
   Verticillium diseases, caused for example by *Verticillium dahliae ;*
Canker, broom and dieback diseases such as :
   Nectria diseases, caused for example by *Nectria galligena ;*
Blight diseases such as :
   Monilinia diseases, caused for example by *Monilinia laxa ;*
Leaf blister or leaf curl diseases such as :
   Exobasidium diseases caused for example by *Exobasidium vexans ;*
   Taphrina diseases, caused for example by *Taphrina deformans* ;
Decline diseases of wooden plants such as :
   Esca diseases, caused for example by *Phaemoniella clamydospora* ;
   Eutypa dyeback, caused for example by *Eutypa lata ;*
   Ganoderma diseases caused for example by *Ganoderma boninense ;*
   Rigidoporus diseases caused for example by *Rigidoporus lignosus ;*
Diseases of Flowers and Seeds such as :
   Botrytis diseases caused for example by *Botrytis cinerea* ;
Diseases of Tubers such as :
   Rhizoctonia diseases caused for example by *Rhizoctonia solani* ;
   Helminthosporium diseases caused for example by *Helminthosporium solani* ;
Club root diseases such as :
   Plasmodiophora diseases, cause for example by *Plamodiophora brassicae ;*
Diseases caused by Bacterial Organisms such as :
   Xanthomonas species for example *Xanthomonas campestris* pv. oryzae ;
   Pseudomonas species for example *Pseudomonas syringae* pv. lachrymans ;
   Erwinia species for example *Erwinia amylovora.*

The composition according to the invention may also be used against fungal diseases liable to grow on or inside timber. The term "timber" means all types of species of wood, and all types of working of this wood intended for construction, for example solid wood, high-density wood, laminated wood, and plywood. The method for treating timber according to the invention mainly consists in contacting one or more compounds according to the invention or a composition according to the invention; this includes for example direct application, spraying, dipping, injection or any other suitable means.

The dose of active compound usually applied in the method of treatment according to the invention is generally and advantageously from 10 to 800 g/ha, preferably from 50 to 300 g/ha for applications in foliar treatment. The dose of active substance applied is generally and advantageously from 2 to 200 g per 100 kg of seed, preferably from 3 to 150 g per 100 kg of seed in the case of seed treatment.

It is clearly understood that the doses indicated herein are given as illustrative examples of the method according to the invention. A person skilled in the art will know how to adapt the application doses, notably according to the nature of the plant or crop to be treated.

The compounds or mixtures according to the invention can also be used for the preparation of composition useful to curatively or preventively treat human or animal fungal diseases such as, for example, mycoses, dermatoses, trichophyton diseases and candidiases or diseases caused by *Aspergillus spp.,* for example *Aspergillus fumigatus.*

The present invention further relates to the use of compounds of the formula (I) as herein defined for the control of phytopathogenic fungi.
The present invention further relates to the use of compounds of the formula (I) as herein defined for the treatment of transgenic plants.
The present invention further relates to the use of compounds of the formula (I) as herein defined for the treatment of seed and of seed of transgenic plants.
The present invention further relates to a process for producing compositions for controlling phytopathogenic harmful fungi, characterized in that derivatives of the formula (I) as herein defined are mixed with extenders and/or surfactants.The various aspects of the invention will now be illustrated with reference to the following table of compound examples and the following preparation or efficacy examples.

Table 1 illustrates in a non-limiting manner examples of compounds of formula (I) according to the invention :

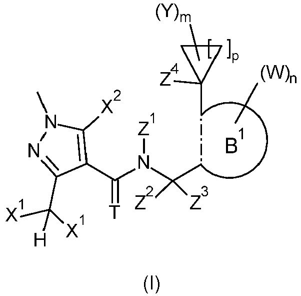

In table 1, unless otherwise specified, M+H (Apcl+) means the molecular ion peak plus 1 a.m.u. (atomic mass unit) as observed in mass spectroscopy via positive atmospheric pressure chemical ionisation.

In table 1, the logP values were determined in accordance with EEC Directive 79/831 Annex V.A8 by HPLC (High Performance Liquid Chromatography) on a reversed-phase column (C 18), using the method described below :
Temperature: 40 °C ; Mobile phases : 0.1% aqueous formic acid and acetonitrile ; linear gradient from 10% acetonitrile to 90% acetonitrile.

Calibration was carried out using unbranched alkan-2-ones (comprising 3 to 16 carbon atoms) with known logP values (determination of the logP values by the retention times using linear interpolation between two successive alkanones). lambda-max-values were determined using UV-spectra from 200 nm to 400 nm and the peak values of the chromatographic signals.

In table 1, "position" denotes the point of attachment of the 1-substitutedcycloalkyl residue to the B¹ heterocyclyl ring based on the IUPAC numbering of heterocyclic rings.

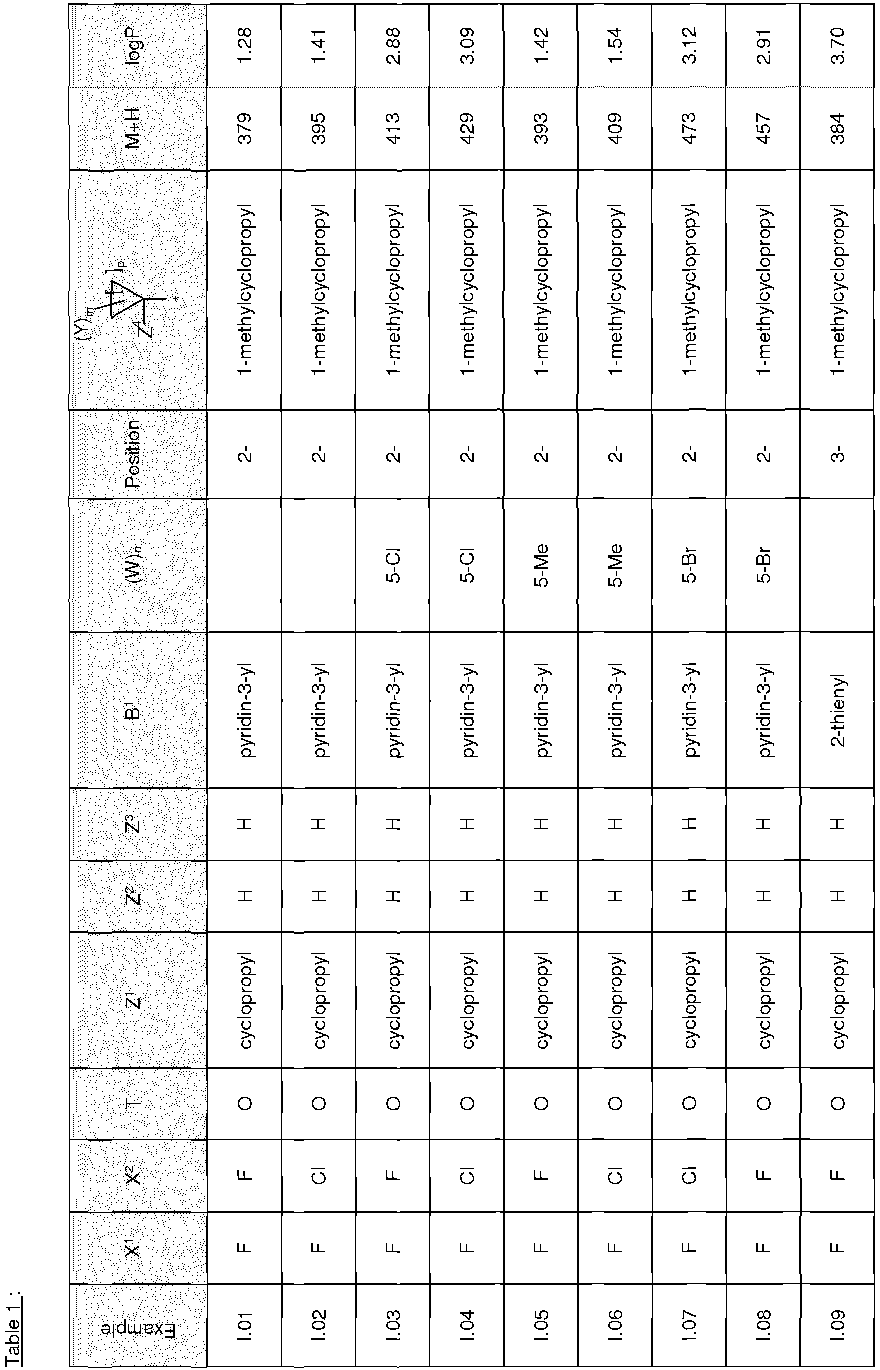

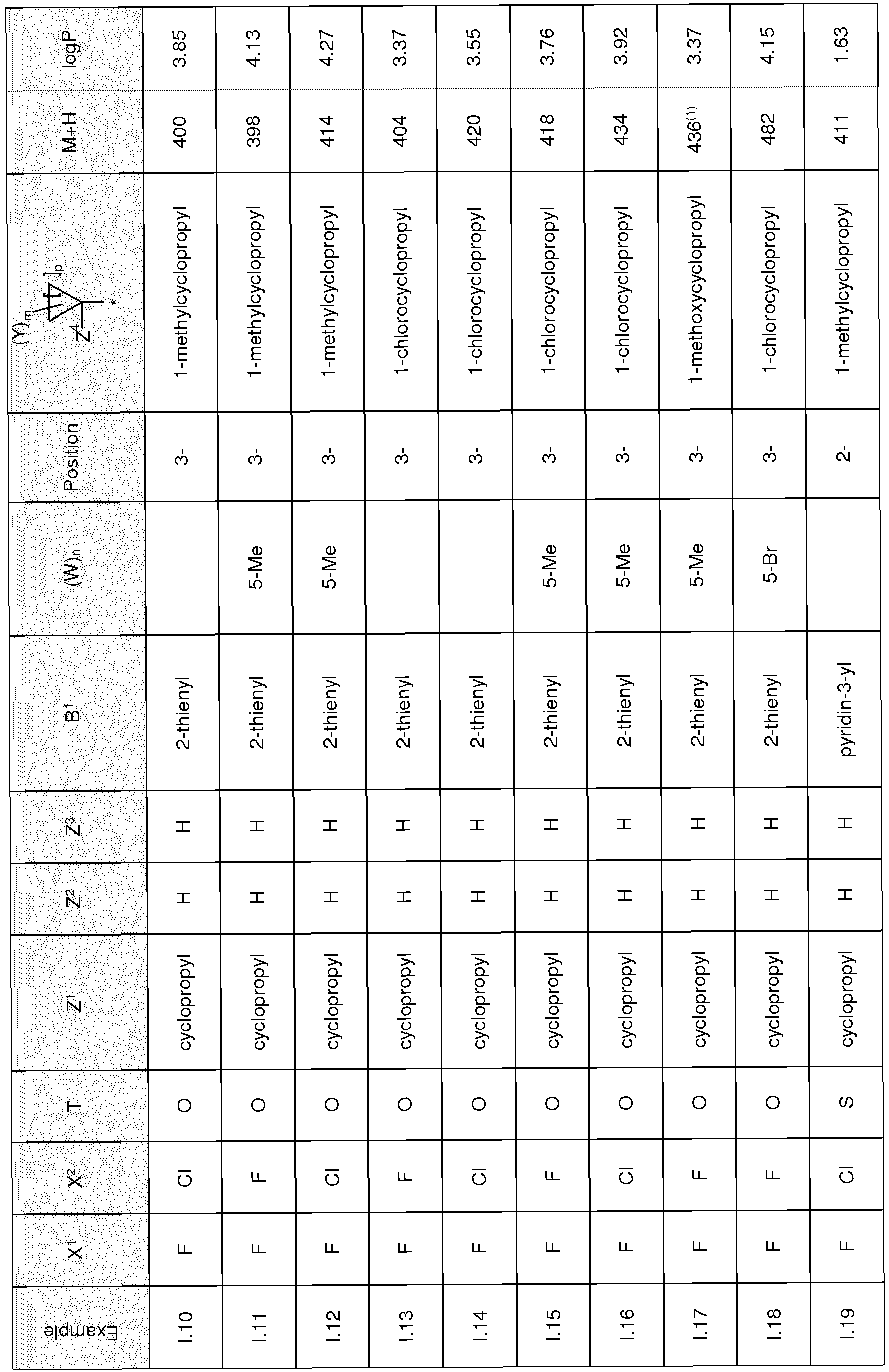

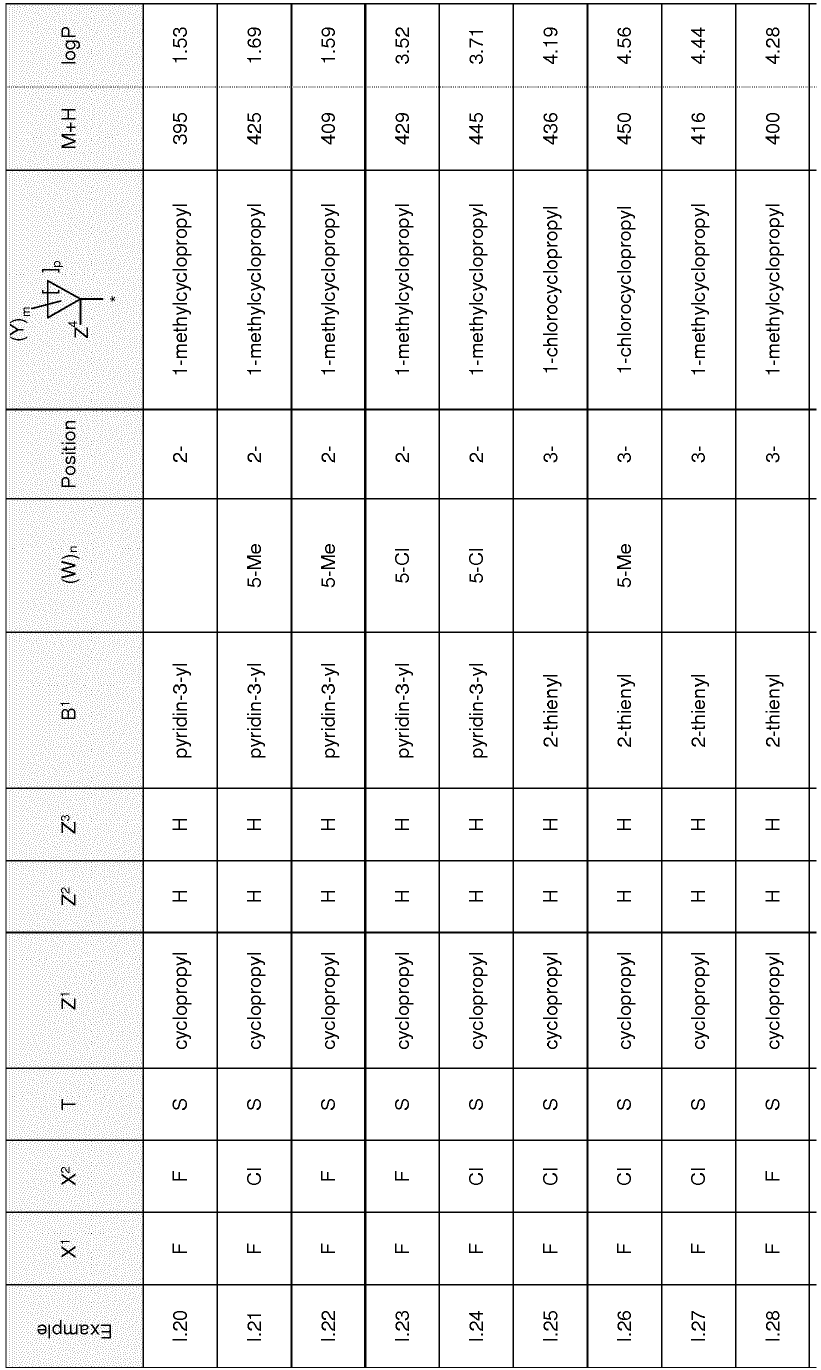

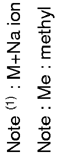

Table 2 illustrates in a non limiting manner examples of compounds of formula (IIa) according to the invention,

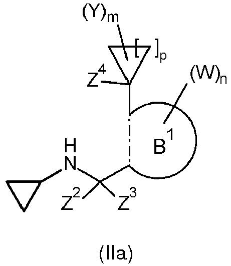

In table 2, M+H (Apcl+) and logP are defined as for table 1.

In table 2, "position" denotes the point of attachment of the 1-substitutedcycloalkyl residue to the B¹ heterocyclyl ring based on the IUPAC numbering of heterocyclic rings.

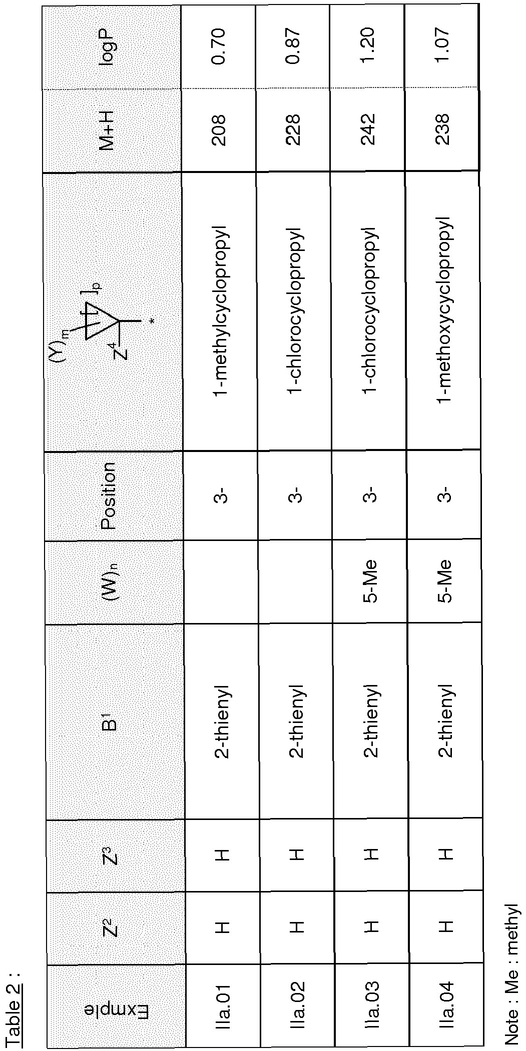

Table 3 illustrates in a non limiting manner examples of compounds of formula (IV) according to the invention,

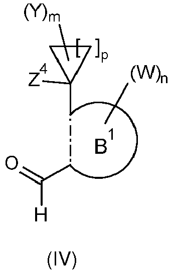

In table 3, M+H (Apcl+) and logP are defined as for table 1.

In table 3, "position" denotes the point of attachment of the 1-substitutedcycloalkyl residue to the B¹ heterocyclyl ring based on the IUPAC numbering of heterocyclic rings.

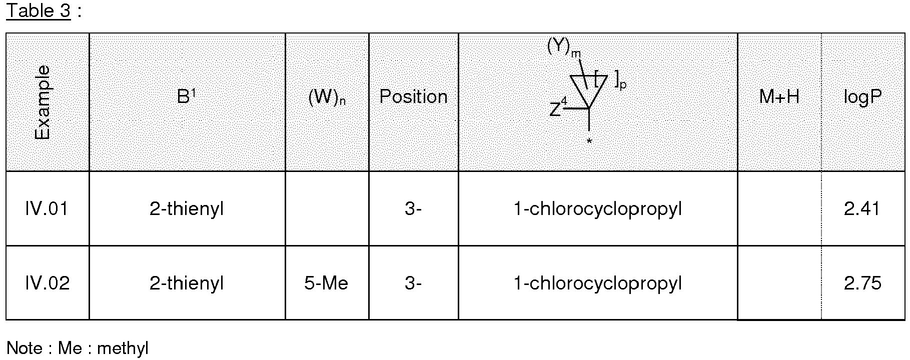

Table 4 illustrates in a non limiting manner examples of compounds of formula (Va1) according to the invention, 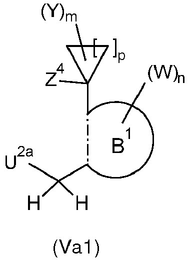 wherein U^{2a} represents a halogen atom.

In table 4, M+H (Apcl+) and logP are defined as for table 1.

In table 4, "position" denotes the point of attachment of the 1-substitutedcycloalkyl residue to the B¹ heterocyclyl ring based on the IUPAC numbering of heterocyclic rings.

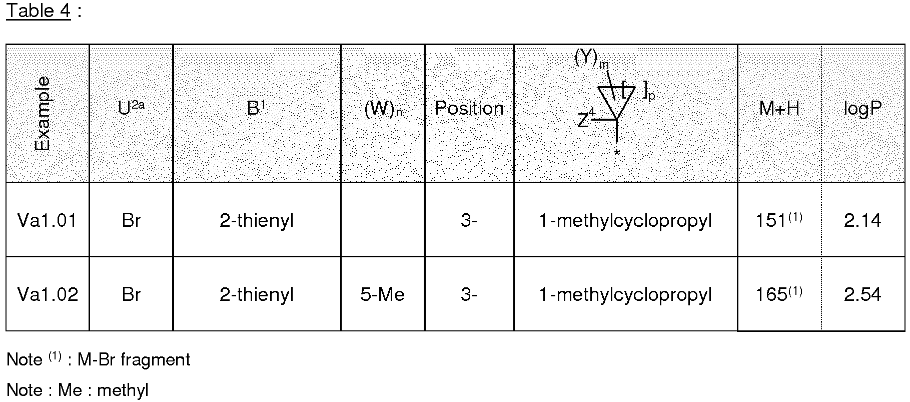

Table 5 illustrates in a non limiting manner examples of compounds of formula (Vc) according to the invention,

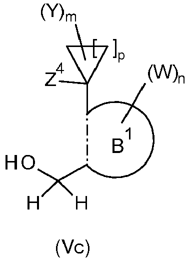

In table 5, M+H (Apcl+) and logP are defined as for table 1.

In table 5, "position" denotes the point of attachment of the 1-substitutedcycloalkyl residue to the B¹ heterocyclyl ring based on the IUPAC numbering of heterocyclic rings.

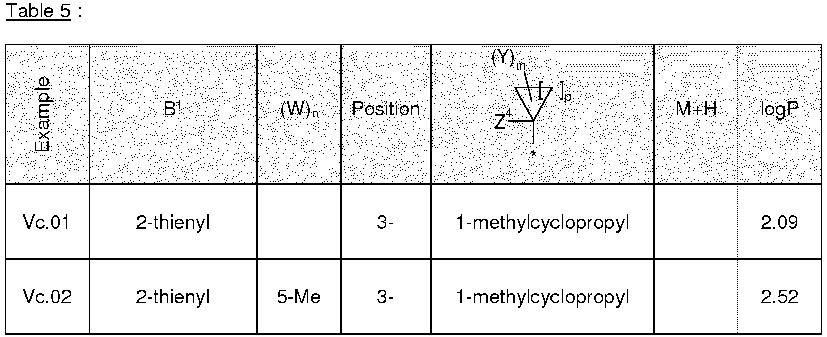

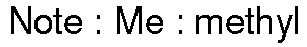

Table 6 provides the NMR data (¹H) of a selected number of compounds from table 1 to table 5.

The ¹H-NMR data of selected examples are stated in the form of ¹H-NMR peak lists. For each signal peak, the δ value in ppm and the signal intensity in brackets are listed.

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.
The ¹H-NMR peak lists are similar to classical ¹H-NMR prints and contain therefore usually all peaks, which are listed at classical NMR-interpretation. Additionally they can show like classical ¹H-NMR prints signals of solvents, stereoisomers of the target compounds, which are also object of the invention, and/or peaks of impurities. To show compound signals in the delta-range of solvents and/or water the usual peaks of solvents, for example peaks of DMSO in d6-DMSO and the peak of water are shown in our ¹H-NMR peak lists and have usually on average a high intensity.
The peaks of stereoisomers of the target compounds and/or peaks of impurities have usually on average a lower intensity than the peaks of target compounds (for example with a purity >90%). Such stereoisomers and/or impurities can be typical for the specific preparation process. Therefore their peaks can help to recognize the reproduction of our preparation process via "side-products-fingerprints".
An expert, who calculates the peaks of the target compounds with known methods (MestreC, ACD-simulation, but also with empirically evaluated expectation values), can isolate the peaks of the target compounds as needed optionally using additional intensity filters. This isolation would be similar to relevant peak picking at classical ¹H-NMR interpretation.
Further details of NMR-data description with peak lists can be found in the publication "Citation of NMR Peaklist Data within Patent Applications" of the Research Disclosure Database Number 564025.

**Table 6 : NMR peak lists**

| |
|---|
| Example I.01: ¹H-NMR (400 MHz, d₆-DMSO): |
| 8.377 (3.3); 8.373 (3.5); 8.365 (3.4); 8.362 (3.2); 7.426 (2.7); 7.409 (3.4); 7.265 (3.2); 7.253 (3.2); 7.246 (2.7); 7.234 (2.5); 7.133 (2.4); 6.998 (5.3); 6.864 (2.7); 4.906 (8.8); 4.039 (0.7); 4.021 (0.7); 3.816 (12.9); 3.309 (43.7); 2.850 (1.0); 2.674 (0.4); 2.670 (0.5); 2.551 (0.4); 2.505 (56.4); 2.501 (73.3); 2.496 (54.1); 2.332 (0.4); 2.328 (0.5); 1.988 (2.9); 1.343 (16.0); 1.297 (0.6); 1.280 (1.1); 1.247 (4.8); 1.193 (0.9); 1.175 (1.6); 1.157 (0.8); 0.920 (6.1); 0.875 (2.2); 0.858 (5.7); 0.841 (2.5); 0.828 (0.5); 0.788 (2.9); 0.778 (6.9); 0.764 (2.2); 0.709 (1.0); 0.692 (4.3); 0.678 (4.3); 0.662 (1.4); 0.539 (4.4); 0.000 (10.1) |
| Example I.02: ¹H-NMR (400 MHz, d₆-DMSO): |
| 8.376 (5.4); 8.368 (5.3); 7.504 (4.9); 7.501 (4.9); 7.485 (5.9); 7.280 (4.1); 7.268 (4.3); 7.260 (3.8); 7.249 (3.3); 7.197 (1.7); 7.063 (3.5); 6.928 (1.7); 4.925 (5.4); 3.914 (16.0); 3.792 (0.4); 3.309 (49.0); 3.285 (0.4); 2.871 (2.3); 2.674 (0.6); 2.670 (0.8); 2.666 (0.6); 2.551 (0.6); 2.505 (90.1); 2.501 (118.1); 2.496 (86.5); 2.332 (0.6); 2.328 (0.8); 2.323 (0.6); 1.988 (0.4); 1.357 (15.7); 1.280 (0.4); 1.246 (0.6); 1.192 (0.5); 1.175 (0.5); 0.935 (7.1); 0.891 (0.6); 0.858 (0.7); 0.840 (0.7); 0.792 (8.0); 0.711 (0.8); 0.667 (0.6); 0.580 (4.6); 0.566 (4.6); 0.486 (5.4); 0.008 (0.8); 0.000 (14.9) |
| Example I.03: ¹H-NMR (500 MHz, CDCl₃): |
| 8.377 (4.9); 8.373 (4.9); 7.411 (4.3); 7.407 (4.3); 7.270 (5.5); 6.942 (1.9); 6.833 (4.0); 6.724 (2.0); 5.296 (0.7); 4.995 (7.6); 4.846 (1.0); 3.845 (16.0); 3.825 (0.5); 2.895 (1.2); 2.374 (1.9); 1.675 (3.9); 1.413 (20.1); 1.394 (2.9); 1.259 (0.4); 1.012 (1.8); 1.001 (6.1); 0.991 (2.6); 0.979 (0.4); 0.890 (0.6); 0.886 (0.5); 0.880 (0.3); 0.875 (0.6); 0.861 (3.2); 0.852 (7.9); 0.849 (7.9); 0.840 (2.3); 0.830 (0.4); 0.821 (0.7); 0.818 (0.7); 0.731 (1.1); 0.719 (3.2); 0.706 (3.0); 0.695 (1.2); 0.682 (0.5); 0.675 (0.6); 0.663 (0.5); 0.651 (1.5); 0.641 (3.7); 0.635 (3.7); 0.619 (0.7); 0.000 (4.4) |
| Example I.04: ¹H-NMR (500 MHz, CDCl₃): |
| 8.377 (10.2); 8.373 (10.1); 8.337 (0.7); 8.333 (0.7); 7.571 (9.6); 7.566 (9.4); 7.265 (19.7); 7.189 (0.6); 7.185 (0.6); 6.878 (1.4); 6.768 (2.8); 6.659 (1.5); 5.296 (1.1); 5.028 (4.7); 4.846 (2.0); 4.826 (0.3); 3.928 (16.0); 3.875 (0.9); 2.904 (2.2); 2.798 (0.4); 2.792 (0.4); 2.784 (0.7); 2.776 (0.4); 2.770 (0.4); 2.374 (3.5); 1.623 (3.3); 1.422 (13.7); 1.393 (6.5); 1.371 (1.0); 1.334 (0.6); 1.324 (0.5); 1.286 (0.7); 1.271 (0.6); 1.258 (1.3); 1.243 (0.4); 1.011 (6.7); 0.990 (3.0); 0.979 (1.6); 0.922 (0.5); 0.895 (0.9); 0.884 (1.5); 0.855 (10.8); 0.830 (2.0); 0.821 (2.3); 0.818 (2.3); 0.809 (1.3); 0.756 (0.6); 0.741 (0.7); 0.730 (1.1); 0.726 (1.1); 0.722 (1.1); 0.708 (0.6); 0.603 (10.7); 0.006 (0.6); 0.000 (16.0); -0.007 (0.9) |
| Example I.05: ¹H-NMR (400 MHz, d₆-DMSO): |
| 8.200 (3.2); 8.196 (3.2); 7.207 (3.3); 7.137 (1.5); 7.002 (3.2); 6.868 (1.6); 4.874 (5.0); 3.819 (7.2); 3.309 (38.7); 2.832 (0.6); 2.670 (0.4); 2.552 (0.3); 2.505 (48.2); 2.501 (62.7); 2.496 (46.3); 2.332 (0.3); 2.328 (0.4); 2.323 (0.3); 2.233 (16.0); 1.988 (0.4); 1.317 (9.8); 1.280 (1.0); 1.246 (4.0); 0.876 (4.7); 0.859 (5.2); 0.841 (2.1); 0.759 (1.9); 0.750 (4.4); 0.735 (1.4); 0.683 (2.5); 0.670 (2.6); 0.543 (2.7); 0.000 (6.5) |
| Example I.06: ¹H-NMR (400 MHz, d₆-DMSO): |
| 8.199 (3.7); 7.302 (3.5); 7.204 (0.9); 7.069 (1.8); 6.935 (0.9); 4.894 (3.2); 3.918 (8.4); 3.309 (26.6); 2.851 (1.2); 2.670 (0.4); 2.665 (0.3); 2.551 (0.4); 2.505 (48.5); 2.501 (63.8); 2.496 (46.8); 2.328 (0.4); 2.323 (0.3); 2.238 (16.0); 1.988 (0.5); 1.331 (8.5); 1.279 (0.6); 1.246 (1.9); 1.193 (0.3); 1.175 (0.5); 0.894 (3.7); 0.876 (1.6); 0.858 (2.4); 0.841 (1.1); 0.811 (0.4); 0.763 (4.0); 0.672 (0.4); 0.580 (2.3); 0.565 (2.3); 0.505 (2.7); 0.000 (8.1); -0.008 (0.4) |
| Example I.07: ¹H-NMR (400 MHz, d₆-DMSO): |
| 8.514 (5.7); 8.508 (6.0); 7.961 (1.9); 7.636 (3.7); 7.223 (1.1); 7.088 (2.2); 6.953 (1.1); 4.917 (4.4); 3.927 (9.8); 3.318 (49.7); 2.899 (16.0); 2.740 (11.9); 2.739 (12.2); 2.678 (0.4); 2.531 (1.0); 2.527 (1.5); 2.518 (19.2); 2.514 (40.8); 2.509 (57.0); 2.504 (42.9); 2.500 (22.2); 2.459 (0.5); 2.454 (0.5); 2.336 (0.4); 1.360 (9.4); 0.944 (4.1); 0.895 (0.4); 0.815 (4.7); 0.620 (2.6); 0.606 (2.7); 0.570 (0.7); 0.522 (3.2) |
| Example I.08: ¹H-NMR (400 MHz, d₆-DMSO): |
| 8.511 (4.7); 8.505 (4.8); 7.960 (1.5); 7.545 (4.5); 7.539 (4.5); 7.148 (2.0); 7.013 (4.7); 6.878 (2.3); 4.898 (8.6); 3.830 (11.5); 3.317 (42.8); 2.898 (12.7); 2.740 (9.6); 2.739 (9.9); 2.678 (0.3); 2.531 (1.0); 2.527 (1.6); 2.518 (17.5); 2.513 (36.2); 2.509 (50.0); 2.504 (36.7); 2.500 (18.7); 2.458 (0.6); 2.336 (0.3); 1.348 (16.0); 0.946 (1.4); 0.931 (4.9); 0.921 (2.2); 0.814 (2.2); 0.804 (5.4); 0.799 (5.4); 0.788 (1.9); 0.730 (0.8); 0.712 (3.3); 0.699 (3.4); 0.695 (3.0); 0.682 (1.2); 0.550 (3.3) |
| Example I.09: ¹H-NMR (400 MHz, d₆-DMSO): |
| 7.294 (6.4); 7.282 (6.7); 7.107 (2.3); 6.972 (5.2); 6.917 (6.8); 6.904 (6.4); 6.837 (2.6); 5.758 (1.4); 4.912 (9.6); 3.804 (14.7); 3.418 (0.5); 3.368 (1.1); 3.318 (138.2); 3.268 (1.5); 2.824 (0.9); 2.531 (0.8); 2.518 (13.5); 2.514 (28.1); 2.509 (38.8); 2.504 (28.5); 2.500 (14.4); 2.464 (0.8); 2.459 (0.8); 2.455 (0.6); 1.272 (16.0); 0.741 (7.3); 0.730 (6.0); 0.725 (4.7); 0.713 (1.7); 0.690 (3.3); 0.682 (5.8); 0.666 (1.9); 0.649 (0.5); 0.638 (0.4); 0.608 (1.1); 0.597 (3.3); 0.590 (3.7); 0.582 (3.0); 0.569 (1.0) |
| Example I.10: ¹H-NMR (400 MHz, d₆-DMSO): |
| 7.296 (6.3); 7.283 (6.6); 7.128 (2.4); 6.994 (5.3); 6.909 (6.6); 6.896 (6.1); 6.859 (2.7); 5.758 (4.2); 4.928 (0.8); 3.893 (16.0); 3.417 (0.4); 3.368 (1.2); 3.318 (191.7); 3.269 (1.0); 2.853 (0.8); 2.678 (0.3); 2.531 (0.5); 2.518 (18.8); 2.513 (39.5); 2.509 (54.4); 2.504 (39.1); 2.500 (18.8); 2.465 (0.4); 2.460 (0.4); 2.336 (0.4); 1.280 (5.0); 0.747 (2.4); 0.691 (4.2); 0.619 (2.1); 0.568 (2.6) |
| Example I.11: ¹H-NMR (300 MHz, CDCl₃): |
| 7.264 (5.4); 7.081 (1.1); 6.899 (2.2); 6.717 (1.1); 6.557 (4.4); 6.554 (4.6); 5.301 (0.9); 4.928 (5.7); 3.810 (13.5); 2.914 (0.8); 2.380 (16.0); 2.008 (7.3); 1.604 (1.1); 1.330 (0.4); 1.280 (15.7); 0.767 (1.5); 0.746 (5.5); 0.725 (2.8); 0.703 (2.2); 0.687 (2.3); 0.672 (3.9); 0.654 (3.9); 0.642 (5.8); 0.636 (4.9); 0.621 (1.8); 0.000 (5.8); -0.011 (0.3) |
| Example I.12: ¹H-NMR (300 MHz, CDCl₃): |
| 7.262 (11.3); 6.952 (0.6); 6.770 (1.3); 6.588 (0.7); 6.549 (4.0); 5.301 (0.9); 4.962 (0.6); 3.932 (0.4); 3.923 (0.6); 3.896 (14.2); 2.958 (0.6); 2.938 (0.6); 2.387 (16.0); 2.009 (6.8); 1.574 (3.9); 1.349 (0.4); 1.283 (5.6); 1.219 (0.6); 0.756 (2.2); 0.646 (7.0); 0.011 (0.4); 0.000 (12.9); -0.011 (0.7) |
| Example I.13: ¹H-NMR (400 MHz, d₆-DMSO): |
| 7.401 (4.7); 7.388 (5.1); 7.116 (2.0); 7.093 (5.0); 7.080 (4.7); 6.981 (4.1); 6.846 (2.1); 5.757 (2.5); 5.002 (10.8); 3.806 (16.0); 3.316 (15.3); 2.898 (1.0); 2.865 (1.2); 2.740 (0.6); 2.509 (14.4); 1.457 (1.4); 1.438 (5.5); 1.425 (2.6); 1.382 (0.7); 1.338 (2.3); 1.324 (5.2); 1.306 (1.6); 0.774 (0.8); 0.756 (3.6); 0.743 (3.8); 0.727 (1.4); 0.613 (4.2) |
| Example I.14: ¹H-NMR (400 MHz, d₆-DMSO): |
| 7.404 (6.2); 7.392 (6.6); 7.142 (2.3); 7.083 (5.9); 7.071 (5.4); 7.008 (4.8); 6.873 (2.5); 5.759 (0.7); 5.016 (1.6); 3.896 (16.0); 3.314 (65.3); 2.877 (1.2); 2.677 (0.4); 2.513 (45.2); 2.508 (59.6); 2.504 (45.2); 2.335 (0.3); 1.453 (4.1); 1.397 (0.5); 1.332 (3.1); 0.633 (2.6); 0.592 (3.4) |
| Example I.15: ¹H-NMR (400 MHz, d₆-DMSO): |
| 7.109 (1.7); 6.974 (3.6); 6.839 (1.8); 6.774 (4.9); 4.919 (8.1); 3.804 (13.7); 3.314 (78.6); 2.849 (0.9); 2.677 (0.4); 2.513 (53.8); 2.509 (71.0); 2.505 (54.6); 2.363 (16.0); 2.335 (0.6); 1.419 (1.2); 1.400 (4.4); 1.386 (2.0); 1.343 (0.6); 1.301 (1.9); 1.287 (4.2); 1.268 (1.1); 0.769 (0.7); 0.751 (3.0); 0.738 (3.1); 0.722 (1.0); 0.610 (3.1); 0.603 (3.4) |
| Example I.16: ¹H-NMR (400 MHz, d₆-DMSO): |
| 7.135 (1.5); 7.000 (3.3); 6.865 (1.7); 6.763 (4.5); 5.759 (1.7); 4.936 (1.0); 3.893 (11.3); 3.314 (69.6); 2.859 (0.7); 2.678 (0.4); 2.513 (45.4); 2.509 (61.9); 2.505 (48.3); 2.367 (16.0); 2.341 (0.6); 2.336 (0.6); 1.413 (2.7); 1.358 (0.4); 1.295 (2.0); 0.626 (1.7); 0.583 (2.3) |
| Example I.17: ¹H-NMR (400 MHz, d₆-DMSO): |
| 7.096 (1.4); 6.961 (3.0); 6.826 (1.5); 6.625 (4.8); 5.758 (1.3); 4.884 (6.3); 3.804 (13.6); 3.313 (36.6); 3.079 (13.2); 2.825 (0.8); 2.509 (31.8); 2.363 (16.0); 1.029 (1.2); 1.014 (4.1); 0.848 (1.6); 0.835 (4.2); 0.820 (1.3); 0.735 (0.7); 0.719 (2.9); 0.705 (2.9); 0.689 (0.9); 0.561 (3.4) |
| |
| Example IIa.01: ¹H-NMR (300 MHz, CDCl₃): |
| 7.246 (0.4); 7.039 (3.8); 7.022 (4.7); 6.886 (4.2); 6.869 (3.5); 4.123 (14.0); 2.296 (0.5); 2.283 (0.8); 2.274 (1.0); 2.262 (1.7); 2.253 (0.7); 2.249 (1.0); 2.241 (0.9); 2.228 (0.5); 1.851 (1.8); 1.287 (16.0); 0.764 (1.0); 0.743 (4.2); 0.732 (2.0); 0.704 (0.7); 0.684 (0.5); 0.656 (2.4); 0.644 (4.8); 0.639 (3.7); 0.633 (2.0); 0.623 (1.5); 0.496 (0.4); 0.474 (1.8); 0.466 (2.7); 0.460 (1.9); 0.452 (1.9); 0.450 (2.1); 0.445 (3.7); 0.441 (3.2); 0.435 (3.1); 0.428 (3.7); 0.423 (3.7); 0.416 (2.0); 0.406 (0.6); 0.392 (0.4); 0.000 (0.4) |
| Example IIa.02: ¹H-NMR (400 MHz, d₆-DMSO): |
| 7.293 (5.4); 7.280 (6.0); 7.034 (6.0); 7.021 (5.5); 4.825 (0.6); 4.097 (16.0); 3.310 (3.9); 2.765 (1.7); 2.509 (26.8); 2.222 (0.7); 2.213 (1.4); 2.205 (1.9); 2.196 (2.7); 2.188 (2.2); 2.181 (2.1); 2.171 (0.9); 1.417 (2.3); 1.398 (7.5); 1.384 (3.7); 1.343 (0.7); 1.324 (0.6); 1.302 (0.4); 1.283 (3.5); 1.269 (7.3); 1.250 (2.5); 0.422 (1.1); 0.406 (5.4); 0.395 (4.6); 0.389 (4.7); 0.380 (2.2); 0.359 (0.8); 0.351 (0.6); 0.342 (0.6); 0.322 (2.0); 0.313 (5.9); 0.305 (6.3); 0.300 (4.3); 0.288 (1.5) |
| Example IIa.03: ¹H-NMR (400 MHz, d₆-DMSO): |
| 6.465 (1.6); 3.774 (1.8); 3.073 (16.0); 2.396 (0.4); 2.271 (8.6); 2.267 (11.0); 2.263 (8.2); 2.100 (5.6); 1.939 (0.4); 1.931 (0.5); 1.922 (0.4); 1.135 (0.6); 1.121 (1.5); 1.116 (1.7); 1.103 (0.9); 1.003 (0.9); 0.990 (1.7); 0.985 (1.5); 0.971 (0.5); 0.155 (0.9); 0.150 (1.3); 0.140 (1.1); 0.134 (1.1); 0.125 (0.5); 0.061 (0.5); 0.052 (1.5); 0.043 (1.5); 0.037 (1.0) |
| Example IIa.04: ¹H-NMR (400 MHz, d₆-DMSO): |
| 6.595 (3.0); 3.959 (3.7); 3.309 (4.7); 3.055 (16.0); 2.512 (12.9); 2.508 (16.8); 2.504 (12.7); 2.347 (10.5); 2.154 (0.6); 2.146 (0.8); 2.138 (1.1); 2.129 (0.8); 2.122 (0.6); 0.990 (1.1); 0.978 (3.6); 0.972 (3.6); 0.961 (1.4); 0.786 (1.4); 0.775 (3.6); 0.769 (3.6); 0.757 (1.0); 0.385 (0.6); 0.374 (1.8); 0.369 (2.6); 0.358 (2.2); 0.352 (2.1); 0.343 (0.9); 0.279 (1.0); 0.270 (2.9); 0.261 (2.8); 0.255 (1.9); 0.244 (0.5) |
| Example IV.01: ¹H-NMR (400 MHz, CDCl₃): |
| 10.515 (15.8); 10.512 (15.3); 7.626 (8.0); 7.624 (7.8); 7.614 (8.3); 7.611 (8.0); 7.261 (16.6); 7.201 (0.4); 7.189 (14.9); 7.176 (14.4); 5.472 (0.7); 2.113 (1.1); 1.643 (0.4); 1.603 (6.6); 1.588 (14.1); 1.584 (15.6); 1.581 (8.3); 1.570 (8.9); 1.564 (0.9); 1.556 (10.6); 1.527 (1.0); 1.421 (1.0); 1.378 (8.9); 1.367 (9.0); 1.364 (16.0); 1.361 (13.6); 1.360 (13.9); 1.345 (6.4); 1.305 (0.4); 1.255 (0.9); 0.007 (0.8); 0.006 (0.6); -0.001 (18.4); -0.009 (0.6) |
| Example IV.02: ¹H-NMR (400 MHz, CDCl₃): |
| 10.398 (9.5); 7.261 (9.1); 6.879 (4.0); 6.877 (4.0); 2.512 (15.7); 2.509 (16.0); 2.500 (0.3); 1.558 (2.3); 1.553 (5.1); 1.544 (4.0); 1.542 (3.8); 1.539 (4.6); 1.536 (2.6); 1.525 (2.6); 1.432 (0.5); 1.343 (2.7); 1.333 (2.5); 1.329 (4.5); 1.326 (3.7); 1.325 (4.0); 1.310 (1.9); 1.256 (0.5); 0.008 (0.4); 0.000 (9.4); -0.009 (0.3) |
| Example Va1.01: ¹H-NMR (300 MHz, CDCl₃): |
| 7.253 (1.0); 7.232 (0.4); 7.211 (2.9); 7.194 (3.3); 7.160 (0.3); 6.872 (3.3); 6.855 (3.0); 4.902 (12.9); 2.354 (1.4); 1.431 (0.4); 1.328 (16.0); 1.300 (0.4); 1.254 (0.4); 0.843 (1.1); 0.822 (4.3); 0.810 (2.0); 0.766 (0.5); 0.760 (0.4); 0.717 (2.1); 0.705 (4.7); 0.699 (3.8); 0.684 (1.4); 0.000 (0.9) |
| Example Va1.02: ¹H-NMR (300 MHz, CDCl₃): |
| 7.280 (0.4); 7.251 (1.9); 7.240 (0.4); 7.232 (1.0); 7.185 (1.4); 7.161 (0.8); 6.530 (3.2); 6.527 (3.2); 5.292 (0.8); 4.901 (0.6); 4.856 (12.6); 4.500 (0.6); 2.422 (0.4); 2.386 (12.6); 2.383 (12.7); 2.354 (4.4); 1.336 (0.4); 1.329 (0.8); 1.305 (16.0); 1.282 (0.8); 1.270 (0.9); 1.258 (0.8); 1.240 (0.4); 1.233 (0.7); 0.893 (0.5); 0.880 (0.3); 0.817 (1.2); 0.801 (3.6); 0.796 (4.2); 0.784 (1.9); 0.733 (0.5); 0.723 (0.5); 0.673 (2.2); 0.661 (4.8); 0.655 (4.1); 0.640 (1.6); 0.000 (1.4) |
| Example Vc.01: ¹H-NMR (400 MHz, d₆-DMSO): |
| 7.243 (2.8); 7.230 (2.9); 6.870 (3.2); 6.857 (2.9); 5.376 (1.7); 5.362 (3.1); 5.348 (1.7); 4.723 (6.8); 4.710 (6.4); 3.313 (14.9); 2.505 (25.1); 2.500 (31.8); 2.496 (23.6); 1.284 (0.5); 1.218 (16.0); 1.150 (0.5); 0.671 (5.1); 0.663 (2.5); 0.651 (1.0); 0.628 (1.0); 0.616 (3.0); 0.608 (5.5); 0.592 (1.4); 0.000 (25.1); -0.008 (1.5) |
| Example Vc.02: ¹H-NMR (300 MHz, d₆-DMSO): |
| 6.544 (3.7); 5.234 (1.3); 5.216 (2.6); 5.198 (1.5); 4.688 (0.3); 4.634 (5.8); 4.616 (5.4); 3.313 (11.2); 2.500 (21.1); 2.329 (14.1); 1.238 (0.8); 1.196 (16.0); 0.864 (0.4); 0.845 (0.4); 0.823 (0.4); 0.644 (5.1); 0.578 (2.8); 0.568 (5.4); 0.551 (1.4); 0.000 (10.0) |

The following examples illustrate in a non-limiting manner the preparation and efficacy of the compounds of formula (I) according to the invention.

### Preparation example 1 : preparation of N-{[3-(1-chlorocyclopropyl)-5-methyl-2-thienyl]methyl}-N-cyclopropyl-3-(difluoromethyl)-5-fluoro-1-methyl-1H-pyrazole-4-carboxamide (compound 1.15)

### Step 1 : N-{[3-(1-chlorocyclopropyl)-5-methyl-2-thienyl]methyl}cyclopropanamine (compound IIa.03)

To a cooled mixture of 960 mg (4.78 mmol) of 3-(1-chlorocyclopropyl)-5-methylthiophene-2-carbaldehyde (compound V.02) and 546 mg (9.57 mmol) of cyclopropylamine in 10 mL of methanol are added 3 g of 3 Å molecular sieves followed by a slow addition of 718 mg (12 mmol) of acetic acid. The reaction mixture is stirred for 90 min at reflux. The reaction mixture is then cooled to room temperature and 4.06 g (18.11 mmol) of sodium cyanoborohydride are slowly added. The reaction mixture is further stirred for 5 h at reflux. The cooled reaction mixture is then filtered over a cake of diatomaceous earth and the cake washed by methanol. Concentration leaves a residue that is dissolved by ethyl acetate, washed by a 1N aqueous solution of sodium hydroxide followed by a saturated aqueous solution of NaCl. The organic phase is dried, concentrated and purified by column chromatography on silica gel (gradient n-heptane/ethyl acetate) to provide 400 mg (33 % yield) of N-{[3-(1-chlorocyclopropyl)-5-methyl-2-thienyl]-methyl}cyclopropanamine. LogP = 1.20. Mass (M+H) = 242.

### Step 2 : preparation of N-{[3-(1-chlorocyclopropyl)-5-methyl-2-thienyl]methyl}-N-cyclopropyl-3-(difluoromethyl)-5-fluoro-1-methyl-1H-pyrazole-4-carboxamide

In a dried Radleys™ vial, a solution of 46 mg (0.21 mmol) of 3-(difluoromethyl)-5-fluoro-1-methyl-1H-pyrazole-4-carbonyl chloride in 2 mL of dry tetrahydrofuran is added to a mixture of 50 mg (0.20 mmol) of N-{[3-(1-chlorocyclopropyl)-5-methyl-2-thienyl]methyl}cyclopropanamine and 0.032 mL (0.22 mmol) of triethylamine in 3 mL of dry tetrahydrofuran. The reaction mixture is stirred at room temperature for 1 h. The reaction mixture is then filtered over a basic alumina cartridge and the cartridge washed by tetrahydrofuran. Concentration and purification of the residue by preparative HPLC (gradient acetonitrile /water + 0.1% HCO₂H) provides 74 mg (81 % yield) of N-{[3-(1-chlorocyclopropyl)-5-methyl-2-thienyl]-methyl}-N-cyclopropyl-3-(difluoromethyl)-5-fluoro-1-methyl-1H-pyrazole-4-carboxamide. LogP = 3.76. Mass (M+H) = 418.

### General preparation example 2 : thionation of amide of formula (I) on Chemspeed™ apparatus

In a 13 mL Chemspeed™ vial is weighted 0.27 mmol of phosphorous pentasulfide (P2S5). 3 mL of a 0.18 M solution of the amide (I) (0.54 mmol) in dioxane is added and the mixture is heated at reflux for two hours. The temperature is then cooled to 80 °C and 2.5 mL of water are added. The mixture is heated at 80 °C for one more hour. 2 mL of water are then added and the reaction mixture is extracted twice by 4 mL of dichloromethane. The organic phase is deposited on a basic alumina cartridge (2 g) and eluted twice by 8 mL of dichloromethane. The solvents are removed and the crude thioamide derivative is analyzed by LCMS and NMR. Insufficiently pure compounds are further purified by preparative LC.

### Example A : in vivo preventive test on Puccinia recondita (brown rust on wheat)

| | | |
|---|---|---|
| Solvent: | 5 % | by volume of Dimethyl sulfoxide |
| | 10 % | by volume of Acetone |
| Emulsifier: | 1 µL | of Tween® 80 per mg of active ingredient |

The active ingredients are made soluble and homogenized in a mixture of Dimethyl sulfoxide/Acetone/ /Tween® 80 and then diluted in water to the desired concentration.

The young plants of wheat are treated by spraying the active ingredient prepared as described above. Control plants are treated only with an aqueous solution of Acetone/Dimethyl sulfoxide/ Tween® 80. After 24 hours, the plants are contaminated by spraying the leaves with an aqueous suspension of Puccinia recondita spores. The contaminated wheat plants are incubated for 24 hours at 20 °C and at 100 % relative humidity and then for 10 days at 20 °C and at 70-80 % relative humidity.

The test is evaluated 11 days after the inoculation. 0 % means an efficacy which corresponds to that of the control plants while an efficacy of 100 % means that no disease is observed.

In this test the following compounds according to the invention showed efficacy between 70 % and 79 % at a concentration of 100 ppm of active ingredient: I.24.

In this test the following compounds according to the invention showed efficacy between 80 % and 89 % at a concentration of 100 ppm of active ingredient: I.01; I.02; I.04; I.06; I.13; I.14; I.16; I.22; I.25; I.26.

In this test the following compounds according to the invention showed efficacy between 90 % and 100 % at a concentration of 100 ppm of active ingredient: I.03; I.05; I.07; I.08; I.09; I.10; I.11; I.12; I.15; I.17; I.23; I.27; I.28.

### Example B : in vivo preventive test on Uromyces appendiculatus (bean rust)

| | | |
|---|---|---|
| Solvent: | 5 % | by volume of Dimethyl sulfoxide |
| | 10 % | by volume of Acetone |
| Emulsifier: | 1 µL | of Tween® 80 per mg of active ingredient |

The active ingredients are made soluble and homogenized in a mixture of Dimethyl sulfoxide/Acetone/ /Tween® 80 and then diluted in water to the desired concentration.
The young plants of bean are treated by spraying the active ingredient prepared as described above. Control plants are treated only with an aqueous solution of Acetone/Dimethyl sulfoxide/ Tween® 80.
After 24 hours, the plants are contaminated by spraying the leaves with an aqueous suspension of *Uromyces appendiculatus* spores. The contaminated bean plants are incubated for 24 hours at 20 °C and at 100% relative humidity and then for 10 days at 20 °C and at 70-80 % relative humidity.
The test is evaluated 11 days after the inoculation. 0 % means an efficacy which corresponds to that of the control plants while an efficacy of 100 % means that no disease is observed.
In this test the following compounds according to the invention showed efficacy between 70 % and 79 % at a concentration of 100 ppm of active ingredient: I.01; I.13; I.24; I.26.
In this test the following compounds according to the invention showed efficacy between 90 % and 100 % at a concentration of 100 ppm of active ingredient: I.03; I.04; I.05; I.06; I.07; I.08; I.09; I.11; I.12; I.15; I.17; I.23; I.28.

Under the same conditions, excellent (at least 90%) to total protection was observed at a dose of 100 ppm of active ingredient with compounds of examples I.11 and I.12, whereas average (less than 70 %) to no protection was observed with compounds CMP1 and CMP2 (isopropyl analogues) disclosed in patent application WO2012/059497 as in table B2:

**Table B2:**

| Example | dose (ppm) | Efficacy |
|---|---|---|
| I.11 from this patent | 100 | 100 |
| CMP1 from WO2012/059497 | 100 | 65 |
| I.12 from this patent | 100 | 100/92 |
| CMP2 from WO2012/059497 | 100 | 0 |

Example CMP1 disclosed in international patent WO2012/059497 corresponds to N-cyclopropyl-3-(difluoromethyl)-5-fluoro-N-[(3-isopropyl-5-methyl-2-thienyl)methyl]-1-methyl-1H-pyrazole-4-carboxamide, and example CMP2 disclosed in international patent WO2012/059497 corresponds to 5-chloro-N-cyclopropyl-3-(difluoromethyl)-N-[(3-isopropyl-5-methyl-2-thienyl)methyl]-1-methyl-1H-pyrazole-4-carboxamide.

These results showed that the compounds according to the invention have a much better biological activity than the structurally closest compounds disclosed in WO2012/059497.

## Claims

1. A compound of formula (I) 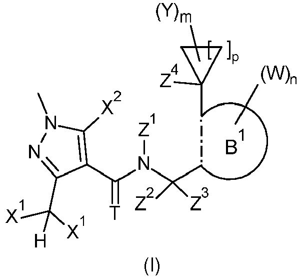 wherein
• X¹ and X² which can be the same or different, represent a chlorine or a fluorine atom,
• T represents O or S;
• n represents 0, 1, 2, 3 or 4;
• m represents 0, 1, 2, 3, or 4;
• p represents 1;
• Z¹ represents a non-substituted cyclopropyl or a cyclopropyl substituted by one or more C₁-C₈-alkyl groups that can be the same or different;
• Z² and Z³, which can be the same or different, represent a hydrogen atom; or non-substituted C₁-C₈-alkyl;
• Z⁴ represents a halogen atom; non-substituted C₁-C₈-alkyl; or non-substituted C₁-C₈-alkoxy;
• B¹ represents a thienyl ring or a pyridinyl ring;
• W independently represents a halogen atom or non-substituted C₁-C₈-alkyl;
• Y independently represents a C₁-C₈-alkyl;
as well as its salts, N-oxides, metal complexes, metalloid complexes and optically active isomers or geometric isomers thereof.

2. A compound according to claim 1 wherein X¹ represents a fluorine atom.

3. A compound according to any one of claims 1 to 2 wherein Z¹ represents a non-substituted cyclopropyl or a cyclopropyl substituted by 1 or 2 C₁-C₅-alkyl;

4. A compound according to any one of claims 1 to 3 wherein Z² and Z³ independently represent a hydrogen atom or a methyl.

5. A compound according to any one of claims 1 to 4 wherein n represents 0, 1 or 2.

6. The compound of formula (IIa) as well as their acceptable salts: 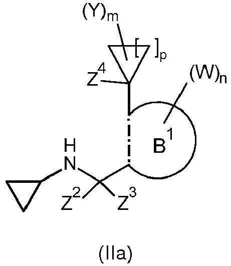 wherein Z², Z³, Z⁴, n, m, p, B¹, W and Y are as defined in any one of claims 1 to 5.

7. The compound of formula (IV) : 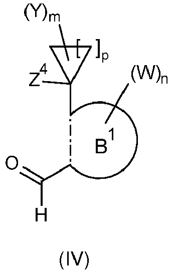 wherein Z⁴, n, m, p, B¹, W and Y are as defined in any one of claims 1 to 5.

8. The compound of formula (Va1) : 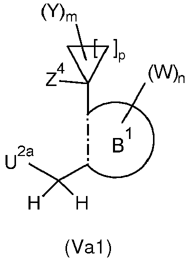 wherein U^{2a} is halogen, and Z⁴, n, m, p, B¹, W and Y are as defined in any one of claims 1 to 5.

9. The compound of formula (Vc) : 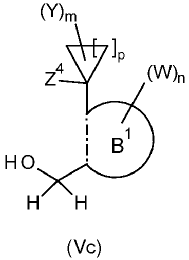 wherein Z⁴, n, m, p, B¹, W and Y are as defined in any ones of claims 1 to 5.

10. A fungicide composition comprising, as an active ingredient, an effective amount of a compound of formula (I) according to claims 1 to 5 and an agriculturally acceptable support, carrier or filler.

11. A method for controlling phytopathogenic fungi of crops, **characterized in that** an agronomically effective and substantially non-phytotoxic quantity of a compound according to any one of claims 1 to 5 or a composition according to claim 10 is applied to the soil where plants grow or are capable of growing, to the leaves and/or the fruit of plants or to the seeds of plants.

12. Use of compounds of the formula (I) according to any one of claims 1 to 5 for the control of phytopathogenic harmful fungi.

13. Process for producing compositions for controlling phytopathogenic harmful fungi, **characterized in that** derivatives of the formula (I) according to any one of claims 1 to 5 are mixed with extenders and/or surfactants.

## Patentansprüche

1. Verbindung der Formel (I) 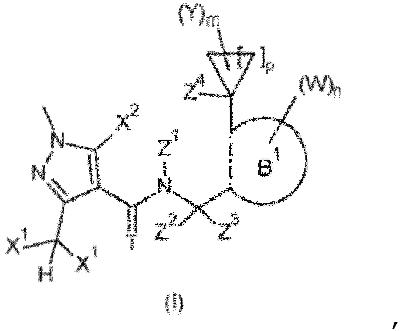 wobei
• X¹ und X², die gleich oder verschieden sein können, für ein Chlor- oder ein Fluoratom stehen,
• T für O oder S steht,
• n für 0, 1, 2, 3 oder 4 steht,
• m für 0, 1, 2, 3 oder 4 steht,
• p für 1 steht,
• Z¹ für unsubstituiertes Cyclopropyl oder durch eine oder mehrere C₁-C₈-Alkylgruppen, die gleich oder verschieden sein können, substituiertes Cyclopropyl steht,
• Z² und Z³, die gleich oder verschieden sein können, für ein Wasserstoffatom oder unsubstituiertes C₁-C₈-Alkyl stehen,
• Z⁴ für ein Halogenatom, unsubstituiertes C₁-C₈-Alkyl oder unsubstituiertes C₁-C₈-Alkoxy steht,
• B¹ für einen Thienylring oder einen Pyridinylring steht,
• W unabhängig für ein Halogenatom oder unsubstituiertes C₁-C₈-Alkyl steht,
• Y unabhängig für C₁-C₈-Alkyl steht,
sowie deren Salze, N-Oxide, Metallkomplexe, Metalloidkomplexe und optisch aktive Isomere oder geometrische Isomere davon.

2. Verbindung nach Anspruch 1, wobei X¹ für ein Fluoratom steht.

3. Verbindung nach einem der Ansprüche 1 bis 2, wobei Z¹ für unsubstituiertes Cyclopropyl oder durch 1 oder 2 C₁-C₅-Alkyl substituiertes Cyclopropyl steht.

4. Verbindung nach einem der Ansprüche 1 bis 3, wobei Z² und Z³ unabhängig für ein Wasserstoffatom oder Methyl stehen.

5. Verbindung nach einem der Ansprüche 1 bis 4, wobei n für 0, 1 oder 2 steht.

6. Verbindung der Formel (IIa) sowie deren unbedenkliche Salze: 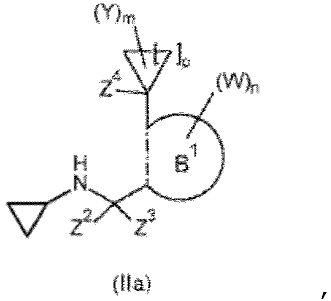 wobei Z², Z³, Z⁴, n, m, p, B¹, W und Y wie in einem der Ansprüche 1 bis 5 definiert sind.

7. Verbindung der Formel (IV): 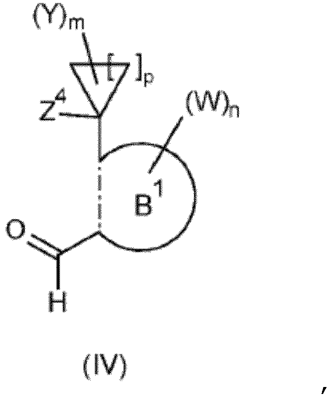 wobei Z⁴, n, m, p, B¹, W und Y wie in einem der Ansprüche 1 bis 5 definiert sind.

8. Verbindung der Formel (Va1): 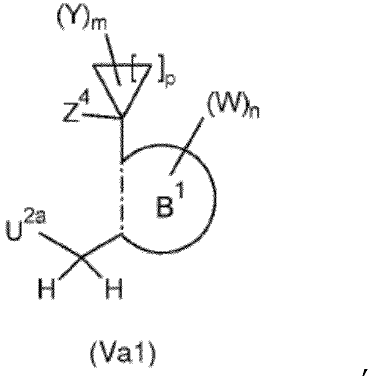 wobei U^{2a} für Halogen steht, und Z⁴, n, m, p, B¹, W und Y wie in einem der Ansprüche 1 bis 5 definiert sind.

9. Verbindung der Formel (Vc): 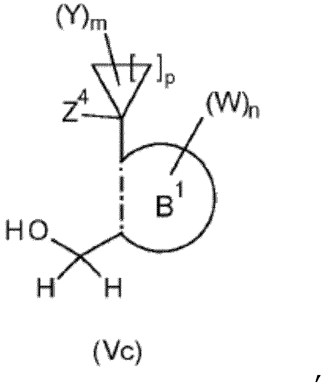 wobei Z⁴, n, m, p, B¹, W und Y wie in einem der Ansprüche 1 bis 5 definiert sind.

10. Fungizidzusammensetzung, umfassend, als Wirkstoff, eine wirksame Menge einer Verbindung der Formel (I) nach Ansprüchen 1 bis 5 und eine landwirtschaftlich unbedenkliche Matrix, einen landwirtschaftlich unbedenklichen Träger oder einen landwirtschaftlich unbedenklichen Füllstoff.

11. Verfahren zur Bekämpfung phytopathogener Pilze auf Kulturpflanzen, **dadurch gekennzeichnet, dass** man eine landwirtschaftlich wirksame und im Wesentlichen nicht phytotoxische Menge einer Verbindung nach einem der Ansprüche 1 bis 5 oder einer Zusammensetzung nach Anspruch 10 auf dem Boden, auf dem die Pflanzen wachsen oder wachsen können, auf die Blätter und/oder die Früchte von Pflanzen oder die Samen von Pflanzen ausbringt.

12. Verwendung von Verbindungen der Formel (I) nach einem der Ansprüche 1 bis 5 zur Bekämpfung phytopathogener Schadpilze.

13. Verfahren zur Herstellung von Zusammensetzungen zur Bekämpfung phytopathogener Schadpilze, **dadurch gekennzeichnet, dass** man Derivate der Formel (I) nach einem der Ansprüche 1 bis 5 mit Streckmitteln und/oder Tensiden mischt.

## Revendications

1. Composé de formule (I) 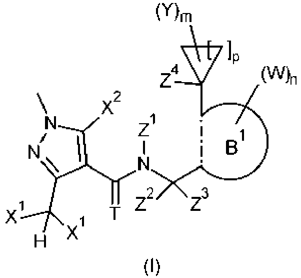 dans lequel
• X¹ et X², qui peuvent être identiques ou différents, représentent un atome de chlore ou de fluor,
• T représente O ou S ;
• n représente 0, 1, 2, 3 ou 4 ;
• m représente 0, 1, 2, 3, ou 4 ;
• p représente 1 ;
• Z¹ représente un cyclopropyle non substitué ou un cyclopropyle substitué par un ou plusieurs groupes alkyle en C₁-C₈ qui peuvent être identiques ou différents ;
• Z² et Z³, qui peuvent être identiques ou différents, représentent un atome d'hydrogène ; ou alkyle en C₁-C₈ non substitué ;
• Z⁴ représente un atome d'halogène ; alkyle en C₁-C₈ non substitué ; ou alcoxy en C₁-C₈ non substitué ;
• B¹ représente un cycle thiényle ou un cycle pyridinyle ;
• W représente indépendamment un atome d'halogène ou alkyle en C₁-C₈ non substitué ;
• Y représente indépendamment un alkyle en C₁-C₈ ;
ainsi que ses sels, N-oxydes, complexes métalliques, complexes de métalloïde et des isomères optiquement actifs ou isomères géométriques de ceux-ci.

2. Composé selon la revendication 1, dans lequel X¹ représente un atome de fluor.

3. Composé selon l'une quelconque des revendications 1 à 2, dans lequel Z¹ représente un cyclopropyle non substitué ou un cyclopropyle substitué par 1 ou 2 groupes alkyle en C₁-C₅.

4. Composé selon l'une quelconque des revendications 1 à 3, dans lequel Z² et Z³ représentent indépendamment un atome d'hydrogène ou un méthyle.

5. Composé selon l'une quelconque des revendications 1 à 4 dans lequel n représente 0, 1 ou 2.

6. Composé de formule (IIa) ainsi que ses sels acceptables : 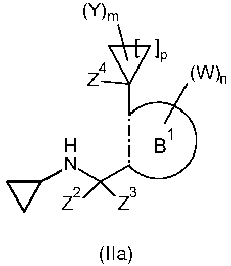 dans lequel Z², Z³, Z⁴, n, m, p, B¹, W et Y sont tels que définis dans l'une quelconque des revendications 1 à 5.

7. Composé de formule (IV) : 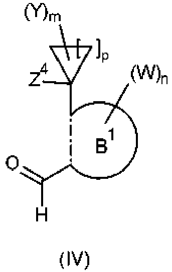 dans lequel Z⁴, n, m, p, B¹, W et Y sont tels que définis dans l'une quelconque des revendications 1 à 5.

8. Composé de formule (Va1) : 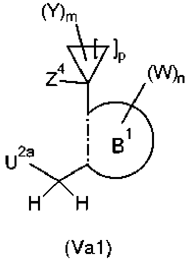 dans lequel U^{2a} est halogène, et Z⁴, n, m, p, B¹, W et Y sont tels que définis dans l'une quelconque des revendications 1 à 5.

9. Composé de formule (Vc) : 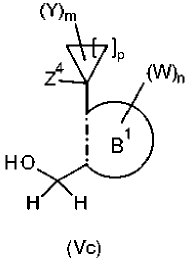 dans lequel Z⁴, n, m, p, B¹, W et Y sont tels que définis dans l'une quelconque des revendications 1 à 5.

10. Composition fongicide comprenant, en tant que substance active, une quantité efficace d'un composé de formule (I) selon les revendications 1 à 5 et un support, un véhicule ou une charge acceptable en agriculture.

11. Procédé de lutte contre des champignons phytopathogènes de cultures, **caractérisé en ce qu'**une quantité agronomiquement efficace et sensiblement non phytotoxique d'un composé selon l'une quelconque des revendications 1 à 5 ou d'une composition selon la revendication 10 est appliquée sur le sol où des plantes croissent ou sont capables de croître, sur les feuilles et/ou les fruits de plantes ou les graines de plantes.

12. Utilisation de composés de formule (I) selon l'une quelconque des revendications 1 à 5 pour la lutte contre des champignons phytopathogènes nuisibles.

13. Procédé de production de compositions pour lutter contre des champignons phytopathogènes nuisibles, **caractérisé en ce que** des dérivés de formule (I) selon l'une quelconque des revendications 1 à 5 sont mélangés avec des matières de charge et/ou des tensioactifs.
